(19) **Europäisches Patentamt European Patent Office Office européen des brevets**

(11) **EP 4 671 367 A1**

(12) **EUROPEAN PATENT APPLICATION**
published in accordance with Art. 153(4) EPC

(43) Date of publication:
31.12.2025 Bulletin 2026/01

(21) Application number: 24756371.1

(22) Date of filing: 27.03.2024

(51) International Patent Classification (IPC):
C12N 5/10 (2006.01)        C07K 16/28 (2006.01)
C07K 19/00 (2006.01)       C12N 15/62 (2006.01)
C12N 15/867 (2006.01)      C12N 5/09 (2010.01)
A61K 39/00 (2006.01)       A61P 35/00 (2006.01)
A61P 35/02 (2006.01)

(86) International application number:
PCT/CN2024/084111

(87) International publication number:
WO 2024/170001 (22.08.2024 Gazette 2024/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 07.04.2023 CN 202310367687

(71) Applicant: Novatim Immune Therapeutics
(Zhejiang) Co., Ltd
Huzhou, Zhejiang 313399 (CN)

(72) Inventors:
• WU, Guoxiang
  Huzhou, Zhejiang 313399 (CN)
• CAI, Xianghai
  Huzhou, Zhejiang 313399 (CN)
• ZHANG, Shun
  Huzhou, Zhejiang 313399 (CN)
• ZHAO, Tao
  Huzhou, Zhejiang 313399 (CN)
• SUN, Luqian
  Huzhou, Zhejiang 313399 (CN)
• ZHANG, Chao
  Huzhou, Zhejiang 313399 (CN)

(74) Representative: Lavoix
Bayerstraße 83
80335 München (DE)

Remarks:
The complete document including Reference
Table(s) and the Sequence Listing(s) can be
downloaded from the EPO website

(54) **BISPECIFIC CAR-T CELLS TARGETING BCMA AND CD19**

(57) Provided are CAR-T cells targeting BCMA and CD19. Specifically, provided is a parallel expression structure for chimeric antigen receptors (CARs) for B cell maturation antigen (BCMA) and a CD19 antigen molecules, and optimizes a CD28 costimulatory domain motif. Further provided is a use of the chimeric antigen receptors for adoptive T cell therapy for B cell-related conditions.

EP 4 671 367 A1

## Description

## Technical field

The present invention is related to the field of biopharmaceutics, and more particularly to a B19 CAR-T cell targeting BCMA and CD19.

## Background

B cell maturation antigen (BCMA), or CD269, is a member of the tumor necrosis factor receptor superfamily member 17 (TNFRS17), which consists of 185 amino acid residues and belongs to the type III transmembrane protein. It comprises a cysteine-rich extracellular domain and has certain conservation. As a cell surface protein expressed only on the B-cell lineage, it, together with 2 other members of TNF receptor superfamily , B-cell activation factor (BAFF) receptor and transmembrane activator and calcium-modulator and cyclophilin ligand (TACI), plays an important regulatory role in the proliferation, survival, maturation and differentiation into plasma cells in B cells. These 3 receptors support the long-term survival of B cells at different developmental stages by binding to BAFF and/or its homologous ligand APRIL. And studies on BCMA-knockout mice further suggest that BCMA, although not essential for overall B cell homeostasis, is critical for the survival of long-lived plasma cells. Meanwhile, BCMA is highly expressed in malignant MM plasma cells and increases with disease progression. Gene and protein expression profiling analysis confirms that BCMA is the most selectively expressed cell surface receptor on MM cell lines, making it a very promising and ideal target for CAR-T cell therapy in MM.

CD19 is a 95 kDa glycoprotein on the surface of B cells that is expressed from the early stage in B cell development until their differentiation into plasma cells. CD19 is a member of the immunoglobulin (Ig) superfamily and participates in the regulation of B cell receptor signaling processes as one of the components of the B cell surface signaling complex. In a CD19-deficient mouse model, B cells in peripheral lymphoid tissues significantly reduces, and the response to vaccines and mitogens also decreases, accompanied by a decrease in serum Ig levels. It is generally accepted that CD19 expression is restricted to the B-cell lineage and is not expressed on the surface of hematopoietic stem cells. CD19 is also expressed on the cell surface of most B-cell lymphoma, mantle cell lymphoma, ALLs, CLLs, hairy cell leukemia, and some the of acute myeloid leukemia. Therefore, CD19 is a valuable immunotherapeutic target in the treatment of leukemia/lymphoma. CD19 is not expressed on the surface of most normal cells, such as pluripotent hematopoietic stem cells. This feature allows CD19 to be used as a safe therapeutic target to minimize the risk of autoimmune disease or irreversible myelotoxic damage in patients. Currently, anti-CD19 antibodies or scFv fragments have been developed and have demonstrated promising applications in mouse models and humans/primates. Pediatric and adult relapsed or refractory acute B-cell lymphomas have an approximately 90% complete remission rate after treatment with CD19 CAR-T cells expressing CD28 or 4-1BB. More recently, CD19 CAR-T cell therapy in diffuse large B-cell lymphoma, follicular lymphoma, or chronic lymphoma exhibits an overall remission rate of 50%-100%. CD19 CAR-T cell therapy for multiple myeloma has clinical advantages due to the fact that terminally differentiated plasma cells do not express CD19 and malignant B-cell precursors continuously produce malignant plasma cells.

In addition, for CD19-negative recurrence and CD19 low-expressing B-cell/plasma cell tumors, problems of mutation-prone, low expression and immune escape that exist with CD19 can be addressed by developing dual-targeted BCMA and CD19 CAR-T cells.

Therefore, there is a need in this field to develop a CAR-T cell that target both BCMA and CD19.

## Summary of the invention

The object of the present invention is to provide a parallel structured CAR-T cell targeting both BCMA and CD19 with modification of the intracellular CD28 co-stimulatory domain, and applications thereof.

The present invention jointly targets dual tumor targets BCMA and CD19, has characteristics of strong specificity and high targeting ability, can effectively improve and prolong therapeutic effects of CAR-T cells, and has better therapeutic effects on multiple myelomas, leukemias, or B-cell lymphomas positive for the surface antigens BCMA and CD19, thereby effectively avoiding off-target escape from a single target, and improving the safety of the CAR-T drug.

In the first aspect of the present invention, is provided an engineered immune cell which expresses a BCMA CAR and a CD19-mutant CAR in parallel, wherein the amino acid sequence of the BCMA CAR is as shown in SEQ ID NO: 15; the amino acid sequence of the CD19-mutant CAR is as shown in SEQ ID NO: 16; wherein the intracellular co-stimulatory domain of the CD19-mutant CAR comprises a CD28 mutant as shown in SEQ ID NO: 14, and the wild-type motif YMNM (SEQ ID NO: 18) is mutated to YMFM (SEQ ID NO: 19) in the CD28 mutant.

CD28-YMFM, the intracellular co-stimulatory domain of the CD19-mutant CAR, is a mutant of wild-type CD28-YMNM, and the amino acid sequence thereof is as shown in SEQ ID NO: 14.

In another preferred embodiment, the immune cell is an NK cell, a macrophage or a T cell, preferably a T cell.

In another preferred embodiment, the BCMA CAR and CD19-mutant CAR are located on cell membrane of the immune cell.

In the second aspect of the present invention, is provided a method for preparing the engineered immune cell of the first aspect of the present invention, comprising steps of:

(A) providing an immune cell to be engineered; and
(B) engineering the immune cell such that the immune cell expresses the BCMA CAR and CD19-mutant CAR, thereby obtaining an engineered immune cells according to the first aspect of the present invention.

In another preferred embodiment, step (A) further comprises isolating and/or activating the immune cells to be engineered.

In another preferred embodiment, step (B) comprises: (B1) transducing a first expression cassette expressing a first CAR targeting BCMA into the immune cell; and (B2) transducing a second expression cassette expressing a second CAR targeting CD19 into the immune cell; wherein step (B1) may be carried out prior to, after, at the same time as, or alternately with step (B2).

In another preferred embodiment, in step (B), the first expression cassette and/or second expression cassette is transduced into the nucleus of the immune cell.

In another preferred embodiment, the engineered immune cell is a NK cell, a macrophage or a T cell.

In another preferred embodiment, the first expression cassette comprises a nucleic acid sequence encoding the BCMA CAR.

In another preferred embodiment, the second expression cassette comprises a nucleic acid sequence encoding the CD19-mutant CAR.

In another preferred embodiment, the first expression cassette and the second expression cassette are located in the same or different vectors.

In another preferred embodiment, the first expression cassette and the second expression cassette are located in the same vector.

In another preferred embodiment, the first expression cassette and the second expression cassette are located in different vectors.

In another preferred embodiment, the vector is a viral vector.

In another preferred embodiment, the vector is selected from a group consisting of DNA, RNA, plasmid, lentiviral vector, adenoviral vector, retroviral vector, transposon, other gene transfer system, and a combination thereof.

In another preferred embodiment, the vector is a lentiviral vector.

In another preferred embodiment, the method further comprises a step of detecting the function and effectiveness of the obtained engineered immune cell.

In the third aspect of the present invention, is provided a pharmaceutical composition comprising the engineered immune cell according to the first aspect of the present invention, and a pharmaceutically acceptable carrier, diluent or excipient.

In another preferred embodiment, the pharmaceutical composition is a liquid preparation.

In another preferred embodiment, the dosage form of the pharmaceutical composition includes an injection.

In another preferred embodiment, the concentration of the engineered immune cell in the pharmaceutical composition is $1\times10^3$-$1\times10^8$ cells/mL, and preferably $1\times10^4$-$1\times10^7$ cells/mL.

In another preferred embodiment, the pharmaceutical composition further comprises other drugs for treating a cancer or tumor (e.g., novel antibody drugs, other CAR-T drugs, or chemotherapy drugs).

In the fourth aspect of the present invention, is provided a use of the engineered immune cell according to the first aspect of the present invention or the pharmaceutical composition according to the third aspect of the present invention, in the preparation of a medicament or formulation for preventing and/or treating a disease, wherein the disease is selected from a group consisting of a hematological tumor, a solid tumor, an autoimmune disease or a combination thereof.

In another preferred embodiment, the hematological tumor is selected from a group consisting of: acute myeloid leukemia, acute lymphoblastic leukemia, acute monocytic leukemia, acute granulocytic leukemia, acute granulo-monocytic leukemia, chronic lymphocytic leukemia, chronic granulocytic leukemia, chronic myelogenous leukemia, lymphoma, Hodgkin lymphoma, non-Hodgkin lymphoma, multiple myeloma (MM), myeloid dysplastic syndrome, POEMS syndrome, systemic amyloidosis, Wahl's macroglobulinemia, monoclonal gammaglobulinemia of unknown origin, and a combination thereof.

In another preferred embodiment, the autoimmune disease is selected from a group consisting of: systemic autoimmune diseases including systemic lupus erythematosus, rheumatoid arthritis, scleroderma, pemphigus, myasthenia gravis, rheumatoid arthritis, desiccation syndrome, and dermatomyositis, etc.; and organ-specific autoimmune diseases including myasthenia gravis, ulcerative colitis, pernicious anemia with chronic atrophic gastritis, pulmonary hemorrhagic nephritis syndrome, pemphigus aspergillosis, primary biliary cirrhosis, multiple cerebrospinal sclerosis, acute idiopathic polyneuritis, optic neuromyelitis optica, inflammatory bowel disease, and a combination thereof.

In another preferred embodiment, the solid tumor is selected from a group consisting of: prostate cancer, liver cancer, head and neck cancer, melanoma, non-Hodgkin's lymphoma, bladder cancer, glioblastoma, cervical cancer, lung cancer, chondrosarcoma, thyroid cancer, renal cancer, mesothelioma, osteosarcoma, cholangiocarcinoma, ovarian cancer, gastric cancer, bladder cancer, meningioma, pancreatic cancer, multiple squamous cell tumor, esophageal cancer, small cell carcinoma of the lung, colorectal cancer, breast cancer, medulloblastoma, breast cancer, nasopharyngeal cancer, thymic cancer, and a combination thereof.

In another preferred embodiment, the hematologic tumor is a multiple myeloma, preferably a recurrent and/or refractory multiple myeloma.

In the fifth aspect of the present invention, is provided a method for inhibiting a tumor cell *in vitro,* which comprises the step:

contacting the tumor cell with the engineered immune cell according to the first aspect of the present invention, or the pharmaceutical composition of the third aspect of the present invention, thereby inhibiting the tumor cell.

In another preferred embodiment, the tumor cell is selected from a group consisting of myeloid leukemia cell, lymphoma cell and myeloma cell.

In another preferred embodiment, the tumor cell is selected from a group consisting of H929 cell, H929-CD19(OE) cell and Nalm6 cell.

In the sixth aspect of the present invention, is provided a method for preventing and/or treating a disease, which comprises:

administering a safe and effective amount of the engineered immune cell according to the first aspect of the present invention, or the pharmaceutical composition of the third aspect of the present invention, to a subject in need thereof.

In another preferred embodiment, the subject includes a human and a non-human mammal.

In another preferred embodiment, the non-human mammal includes a rodent (such as a mouse, a rat, a rabbit), a primate (such as a monkey).

In another preferred embodiment, the method further comprises administering other drugs for treating a cancer or tumor to the subject in need thereof.

In another preferred embodiment, the other drugs includes CAR-T drugs.

In another preferred embodiment, the disease is a cancer or tumor.

In another preferred embodiment, the tumor includes a tumor highly expressing CD19 and/or BCMA.

In another preferred embodiment, the tumor includes a tumor simultaneously expressing CD19 and BCMA.

In another preferred embodiment, the tumor highly expressing CD19 and/or BCMA is a multiple myeloma, preferably a recurrent and/or refractory multiple myeloma.

In the seventh aspect of the present invention, is provided a fusion protein which comprises a BCMA CAR sequence and a CD19-mutant CAR sequence connected by a self-cleaving sequence, wherein the amino acid sequence of the BCMA CAR is as shown in SEQ ID NO: 15; the amino acid sequence of the CD19-mutant CAR is as shown in SEQ ID NO: 16; wherein the intracellular co-stimulatory domain of the CD19-mutant CAR comprises a CD28 mutant as shown in SEQ ID NO: 14, and the wild-type motif YMNM is mutated to YMFM in the CD28 mutant.

In another preferred embodiment, the self-clipping sequence is a T2A sequence.

In the eighth aspect of the present invention, is provided a polynucleotide encoding the fusion protein according to the seventh aspect of the present invention.

In another preferred embodiment, the polynucleotide is DNA or RNA.

In the ninth aspect of the present invention, is provided a vector comprising the polynucleotide according to the eighth aspect of the present invention.

In another preferred embodiment, the vector is selected from the group consisting of plasmid, viral vector, transposon and a combination thereof.

In another preferred embodiment, the vector is selected from a group consisting of lentiviral vector, adenoviral vector, adeno-associated viral vector and a combination thereof.

In another preferred embodiment, the vector comprises one or more promoters, wherein the promoter is operably linked to the nucleic acid sequence, enhancer, intron, transcription termination signal, polyadenylation sequence, replication origin, selective marker, nucleic acid restriction site, and/or homologous recombination site.

It should be understood that, within the scope of the present invention, the technical features specifically described above and below (such as the Examples) can be combined with each other, thereby constituting a new or preferred technical solution which needs not be described one by one.

**DESCRIPTION OF DRAWINGS**

Figure 1 shows the structural schematic diagrams of B19 CAR, B19-wildtype CAR, BCMA CAR, CD19-mutant CAR and CD19-wildtype CAR.

Figure 2 shows the profile of B19 CAR plasmid.

Figure 3 shows the profile of B19-wildtype CAR plasmid.

Figure 4 shows the profile of CD19-mutant CAR plasmid.

Figure 5 shows the profile of CD19-wildtype CAR plasmid.

Figure 6 shows the profile of BCMA CAR plasmid.

Figure 7 shows the detection results of CD19 antigen and BMCA antigen on the membrane surface of 7 tumor cells.

Figure 8 shows the mechanism diagram of B19 CAR-T killing BCMA+ or/and CD19+ tumor cells.

Figure 9 shows the experimental flow chart.

Figure 10 shows the expansion curves of the 4 CAR-T cells before repeated stimulation, wherein A, B, and C correspond to donors A, E, and C, respectively.

Figure 11 shows the CAR-T cell viability at the preparation stage (Donor C).

Figure 12 shows the detection results of CAR-T preparation success rate (A) and CAR-T positive rate (B).

Figures 13A and 13B show the CAR positivity rates of the 5 types of CAR-T cells at the preparation stage. Figures 13A and B correspond to donors A and B, respectively.

Figure 14 shows the CAR positivity rates before repeated stimulation (A) and after 3 rounds of repeated stimulation with H929-luc+Nalm6-luc (B).

Figure 15 shows the CAR-T cell subtypes before repeated stimulation (A), after 3 rounds of repeated stimulation with H929-luc+Raji-luc (B) and after 3 rounds of repeated stimulation with H929-luc+Nalm6-luc (C).

Figure 16 shows the percentage of CD4+ and CD8+ T cells before repeated stimulation (A) and after 3 rounds of repeated stimulation with H929-luc + Nalm6-luc (B).

Figure 17 shows the expression percentage of inhibitory receptors Tim-3 and LAG-3 in CAR-T cells after 3 rounds of repeated stimulation with different tumor cells.

Figure 18 shows the expansion curves of four types of CAR-T cells after repeated stimulation. (A) repeated stimulation with H929-luc, Donor A; (B) repeated stimulation with H929-luc+ Nalm6-luc, Donor A; (C) repeated stimulation with H929-luc+ Nalm6-luc, Donor E; (D) repeated stimulation with H929-CD19(OE)-luc, Donor E.

Figure 19 shows killing effects of CAR-T cells co-incubated with multiple tumor cell lines before repeated stimulation (A) and after 2 rounds of Nalm6-luc repeated stimulation (B) at different effector-to-target ratios.

Figures 20A and 20B show CAR-T cytokine release before repeated stimulation (A) and after (B) repeated stimulation (Donor A).

Figures 21A and 21B show CAR-T cytokine release before repeated stimulation (A) and after (B) repeated stimulation (Donor B).

Figure 22 shows the changes of fluorescence values in tumor-bearing mice in the pharmacodynamic assay of CD19-mutant CAR-T and CD19-wildtype CAR-T cells conducted on Nalm6-luc tumor-bearing models (dose: 1.50E+6 cells/mouse).

Figure 23 shows the changes of fluorescence values in tumor-bearing mice in the pharmacodynamic assay of B19 CAR-T and B19-wildtype CAR-T cells conducted on Nalm6-luc tumor-bearing models (dose: 3.00E+6 cells/mouse).

Figure 24 shows the changes of fluorescence values in tumor-bearing mice in the pharmacodynamic assay of B19 CAR-T and B19-wildtype CAR-T cells conducted on Nalm6-luc+H929-luc tumor-bearing models (dose: 3.00E+6 cells/mouse).

Figure 25 shows the changes of fluorescence values in tumor-bearing mice in the pharmacodynamic assay of B19 CAR-T and B19-wildtype CAR-T cells conducted on Nalm6-luc+H929-luc tumor-bearing models (dose: 1.50E+6 cells/mouse).

## EMBODIMENTS

Through extensive and intensive research and massive screening, the present inventors have constructed a B19 CAR-T cell expressing both BCMA CAR and CD19-mutant CAR. In the present invention, a T cell expressing the dual-target B19 CARs was constructed so as to address the problems of therapeutic scope, immune escape and safety in the existing single-target CAR-T immunotherapy. The dual-target B19 CAR-T cell is characterized as follows: (1) using lentivirus to transduce the activated T cells to allow the parallel expression of the BCMA CAR and the CD19-mutant CAR; (2) in the intracellular co-stimulatory domain of the CD19-mutant CAR, mutating the asparagine residue (N) in YMNM motif of the commonly used wild-type CD28 intracellular co-stimulatory domain to a phenylalanine (F) residue. The present invention prepared the CAR-T cell and evaluated functions of the CAR-T *in vitro* and in mice. On this basis, the present invention has been completed.

**Terms**

To understand the disclosure easier, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

The term "antibody" (Ab) shall include, but is not limited to, an immunoglobulin which specifically binds an antigen and comprises at least two heavy (H) chains and two light (L) chains interconnected by disulfide bonds, or an antigen-binding portion thereof. Each H chain comprises a heavy chain variable region (abbreviated herein as VH) and a heavy chain constant region. The heavy chain constant region comprises three constant structural domains CH1, CH2 and CH3.

It should be understood that the names of amino acids herein are represented by the internationally accepted single English letter, to which the corresponding three English letter names of amino acid are as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y) and Val (V).

When referring to amino acid or nucleotide sequences, the term "sequence identity" refers to a measure of the degree of identity between two amino acid or nucleotide sequences (e.g., a query sequence and a reference sequence) and is usually expressed as a percentage. Typically, before calculating the identity percentage between two amino acid or nucleotide sequences, a sequence alignment is performed and a gap (if any) is introduced. For the purposes of the present invention, the open alignment software BLAST (available at the web page ncbi.nlm.nih.gov) can be employed to obtain the optimal sequence alignment by using default settings and calculate the sequence identity between the two amino acid or nucleotide sequences. In some embodiments, the "at least 90% sequence identity" described herein includes, but is not limited to, situations of at least 95%, at least 98%, at least 99%, or even 100% sequence identity.

**BCMA**

BCMA, known as B-cell maturation antigen, also known as CD269, is a member of the TNF receptor superfamily and is expressed only on the surface of mature B cells, not in either T cells or monocytes, and is an important B-cell biomarker. BCMA expression on multiple myeloma cells is significantly higher than that on healthy plasma cells.

**CD19**

CD19 is a 95 kDa glycoprotein on the surface of B-cells. It is commonly believed that CD19 expression is restricted to B-cell lineage, such as the surface of tumor cells such as most B-cell lymphoma, condylomatous lymphoma, and a portion of acute myeloid leukemia cells. Some recent studies have found that plasma cells from patients with multiple myeloma also express low level of CD19, making CD19 a very valuable target for immunotherapy.

**Chimeric antigen receptor (CAR)**

As used herein, a "chimeric antigen receptor (CAR)" is a fusion protein comprising an extracellular domain capable of binding an antigen, a transmembrane domain derived from a different polypeptide from the extracellular domain, and at lease one intracellular domain.

Particularly, the chimeric antigen receptor (CAR) of the present invention comprises the three domains mentioned above. The extracellular domain comprises a target-specific binding element (also known as an antigen binding domain). The intracellular domain includes a co-stimulatory signaling region and a ζ chain. The co-stimulatory signaling region refers to a part of the intracellular domain that includes a co-stimulatory molecule. The co-stimulatory molecule is a cell surface molecule required for efficient response of lymphocytes to antigens, rather than an antigen receptor or its ligand.

A linker (or linking peptide) can be incorporated between the extracellular domain and the transmembrane domain of the CAR, or between the cytoplasmic domain and the transmembrane domain of the CAR.

As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids. Preferably, the linker is a flexible linker, for example, the linker is $(G4S)_n$, wherein n is 1-4.

A preferred embodiment of the present invention comprises two CARs, a BCMA CAR and a CD19-mutant CAR. When the CAR of the present invention is expressed in a T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains from the co-stimulatory molecules and the ζ chains. Preferably, the antigen binding domain is fused with an intracellular domain consisting of a 4-1BB and/or CD28 co-stimulatory domain and a CD3ζ signaling domain. As used herein, "an antigen binding fragment" or "a single antibody domain" refers to a Fab fragment, a Fab' fragment, a F(ab')2 fragment, or a single Fv fragment that has antigen-binding activity. An Fv antibody comprises a heavy chain variable region and a light chain variable region, but does not comprise a constant region, and is the smallest antibody fragment with all antigen binding sites. Generally, an Fv antibody also comprises a polypeptide linker between the VH and VL domain, and can form the structure required for antigen binding. The antigen-binding structural domain is typically a scFv (single-chain variable fragment). ScFvs are typically 1/6 the size of an intact antibody. A single-chain antibody is preferably a sequence of a single amino acid chain encoded by a single nucleotide chain. As a preferred embodiment of the present invention, the scFv comprises an antibody, preferably a single chain antibody, that specifically recognizes the tumor highly expressed antigens CD19 and/or BCMA.

For the hinge region and the transmembrane region (transmembrane domain), the CAR may be designed to include a transmembrane domain fused to the extracellular structural domain of the CAR.

As used herein, "B19 CAR-T" refers to a bispecific T cell targeting BCMA and CD19 of the first aspect of the present invention. In the CAR of the present invention, the CD28 co-stimulatory domain is mutant, wherein in the mutant CD28 co-stimulatory domain, the asparagine residue (N) in the YMNM motif is mutated to a phenylalanine (F) residue.

**Vector**

The present invention also provides a DNA construct encoding CAR sequences of the present invention.

The nucleic acid sequences coding for the desired molecules can be obtained using recombinant methods known in the art. Alternatively, the gene of interest can be produced synthetically.

The present invention also provides a vector in which the expression cassette of the present invention is inserted. Vectors derived from retroviruses such as lentiviruses are suitable tools for achieving long-term gene transfer.

The expression vector constructs of the present invention may also be used for nucleic acid immune and gene therapy, using standard gene delivery protocols. Methods for gene delivery are known in the art. See, e.g., U.S, Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the vector includes, but is not limited to a plasmid, a phagemid, a phage derivative, an animal virus, and a cosmid. Vectors of particular functions include expression vectors, replication vectors, probe generation vectors, and sequencing vectors.

Further, the expression vector may be provided to a cell in the form of a viral vector. Viral vector technology is well known in the art and is described, for example, in Sambrook et al, (2001, Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. Currently, a number of viral based systems have been developed for transferring a gene into mammalian cells. For example, retroviruses provide a convenient platform for gene delivery systems. A selected gene can be inserted into a vector and packaged in retroviral particles using techniques known in the art. In some embodiments, adenovirus vectors are used. In some embodiments, lentivirus vectors are used.

In general, a suitable vector contains an origin of replication functional in at least one organism, a promoter sequence, convenient restriction endonuclease sites, and one or more selectable markers, (e.g., WO 01/96584; WO 01/29058; and U.S, Pat. No.6,326,193).

One example of a suitable promoter is the immediate early cytomegalovirus (CMV) promoter sequence. This promoter sequence is a strong constitutive promoter sequence capable of driving high levels of expression of any nucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter.

Further, the present invention should not be limited to the use of constitutive promoters. Inducible promoters are also contemplated as a part of the present invention. The use of an inducible promoter provides a molecular switch capable of turning on expression of the polynucleotide sequence which it is operatively linked when such expression is desired, or turning off the expression when expression is not desired.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Biological methods for introducing a polynucleotide of interest into a host cell include the use of DNA and RNA vectors. Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes.

Biological methods for introducing a polynucleotide into a host cell include the use of viral vectors. Viral vectors, and especially retroviral vectors, have become the most widely used method for inserting genes into mammalian cells, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

In a preferred embodiment of the present invention, the vector is a lentiviral vector.

The DNA construct further comprises a signal peptide coding sequence. Preferably, the signal peptide sequence is attached upstream of the nucleic acid sequence of the antigen-binding domain.

**Therapeutic application**

In the present invention, is provided a method for stimulating a T cell-mediated immune response to a target cell population or tissue in a mammal, which comprises a step of administering to the mammal a CAR-T cell of the present invention.

In one embodiment, the present invention comprises a cell therapy, wherein T cells are genetically modified to express the CAR of the present invention and the CAR-T cells are infused into a subject in need thereof. The infused cells can kill tumor cells in the body of the subject. Unlike antibody therapy, CAR-T cells are able to replicate *in vivo* and result in a long-term persistence that can lead to sustained tumor control.

In one embodiment, the CAR-T cells of the present invention can undergo robust *in vivo* T cell expansion and can persist for an extended period. In addition, the CAR mediated immune response may be part of an adoptive immunotherapy approach in which CAR-modified T cells induce an immune response specific to the antigen binding moiety in the CAR.

Although the data disclosed herein specifically disclose lentiviral vector comprising anti-CD19/BCMA scFv, hinge and transmembrane domains, and co-stimulatory domain and CD3ζ signaling domain, it is contemplated that the present invention include any number of variations for each of the components of the construct as described elsewhere herein.

Indications that can be treated include CD19 and/or BCMA-associated cancers or tumors, such as CD19 and/or BCMA-positive tumors or cancers. CD19 and/or BCMA-associated cancers or tumors may include solid tumors, in particular hepatocellular cancer, melanoma, ovarian cancer, squamous cell carcinoma of the lung, gastric cancer, breast cancer, or a combination thereof.

Types of cancers treated with the CAR of the present invention include, but are not limited to, carcinomas, embryonal cell tumors, sarcomas, melanomas, and certain benign tumors. They can be classified into adult tumors/-cancers and pediatric tumors/cancers according to patient age.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer and ovarian cancer, etc.

The CAR-modified T cells of the present invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cells into a mammal: i) expanding the cells, ii) introducing a nucleic acid encoding a CAR to the cells, and/or iii) cryopreservation of the cells.

In addition to using a cell-based vaccine in terms of *ex vivo* immunization, the present invention also provides compositions and methods for *in vivo* immunization to elicit an immune response directed against an antigen in a patient.

Typically, activated and expanded cells as described herein may be used to treat and prevent diseases arising in immunocompromised individuals. In particular, the CAR-modified T cells of the present invention are used in the treatment of tumors. In certain embodiments, the cells of the present invention are used to treat patients at risk of developing tumors. Accordingly, the present invention provides methods of treating or preventing a tumor, comprising administering a therapeutically effective amount of the CAR-modified T cell of the present invention to a subject in need thereof.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an effective amount", "an anti-tumor effective amount", "an tumor-inhibiting effective amount", or "a therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of $10^4$ to $10^9$ cells/kg body weight, preferably $10^5$ to $10^6$ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein or other methods known in the art for expanding T cells to therapeutic levels are administered to a patient in combination (e.g., prior to, concurrently with, or subsequent to) any number of related therapeutic forms, wherein the therapeutic forms include, but are not limited to, treatments with the following agents: combination of other prophylactic and/or therapeutic agents for the treatment of tumors (particularly multiple myeloma), other prophylactic and/or therapeutic compounds that can be administered concurrently with the primary active ingredient, or even concurrently in the same composition.

**Main advantages of the present invention include:**

a) The preparation success rate of the B19 CAR-T of the present invention with a mutated intracellular co-stimulation domain CD28 is higher than that of the B19-wildtype CAR-T, which better meets the acute needs of patients.

b) The expansion of the B19 CAR-T of the present invention with a mutated intracellular co-stimulation domain CD28 is superior to that of B19-wildtype CAR-T, CD19-mutant CAR-T and CD19-wildtype CAR-T in the preparation stage and after repeated stimulation with tumor cells.

c) The B19 CAR-T of the present invention kills more types of tumor cells. The B19 CAR-T of the present invention can kill BCMA+ single-positive, CD19+ single-positive and CD19+ BCMA+ double-positive tumor cells, which expands the therapeutic range of CAR-T cells, reduces the risk of immune escape due to down-regulation or deficiency of antigen expression during the treatment of CAR-T cells recognizing a single-target, and enhances the therapeutic effect of B19 CAR-T. The *in vivo* pharmacodynamic test shows that the B19 CAR-T could effectively clear the above tumor cells.

d) The B19 CAR-T of the present invention is superior to the B19-wildtype CAR-T in killing tumor cells Nalm6-luc, H929-luc, MM1S-luc and Nalm6-luc+H929-luc.

e) The B19 CAR-T of the present invention is superior to CD19-mutant CAR-T and CD19-wildtype CAR-T in killing CD19+BCMA-tumor cell Nalm6-luc.

f) After co-incubation with tumor cells, the release of cytokines IL-2, TNF-$\alpha$ and IFN-$\gamma$ of the B19 CAR-T of the present invention with a mutated co-stimulatory domain CD28 is significantly higher than that of the B19-wildtype CAR-T, the CD19-mutant CAR-T, the CD19-wildtype CAR-T and the BCMA CAR-T.

(g) Mutation of the co-stimulatory domain CD28 in of the present invention unexpectedly improves the anti-tumor effect of single-target CD19-mutant CAR-T and dual-target B19 CAR-T in mice, and improves the safety of B19 CAR-T.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

**Example 1 Construction of plasmid**

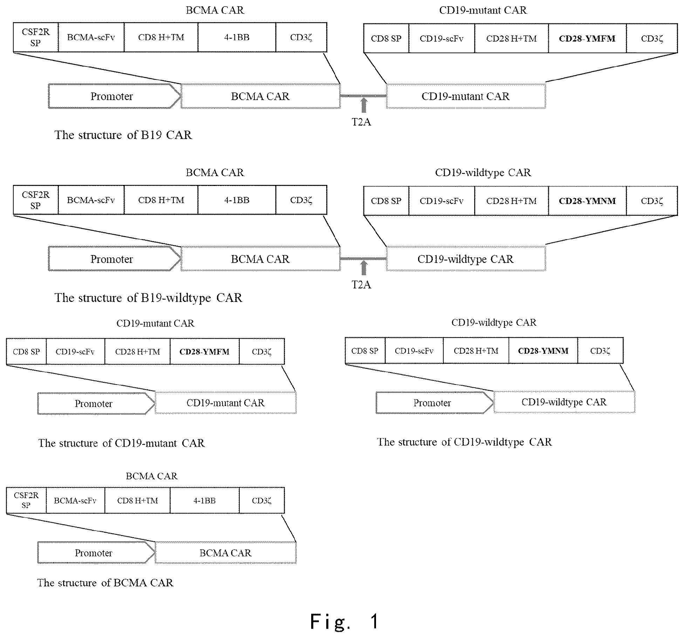

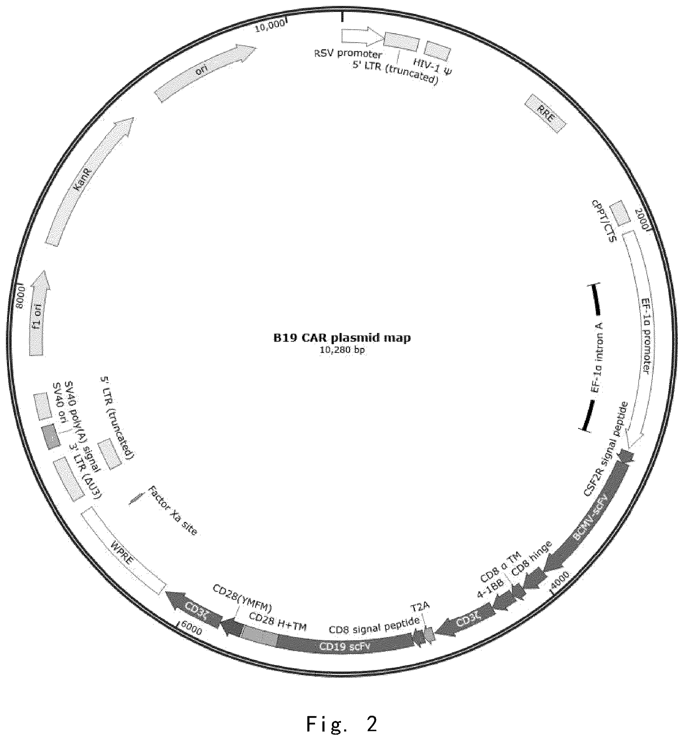

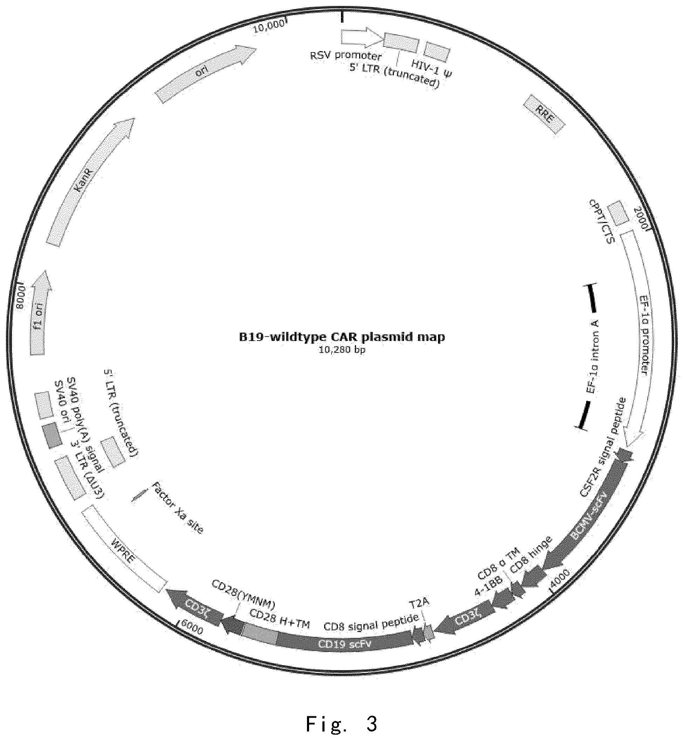

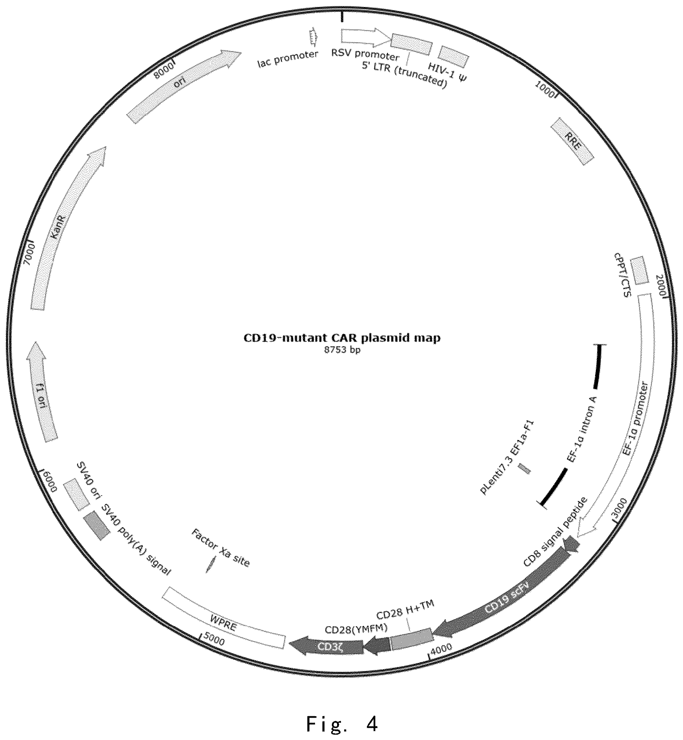

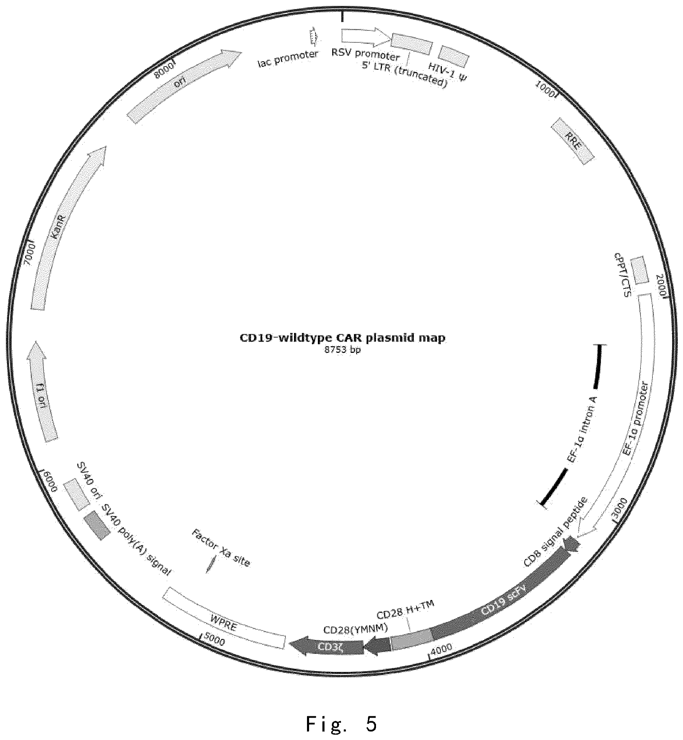

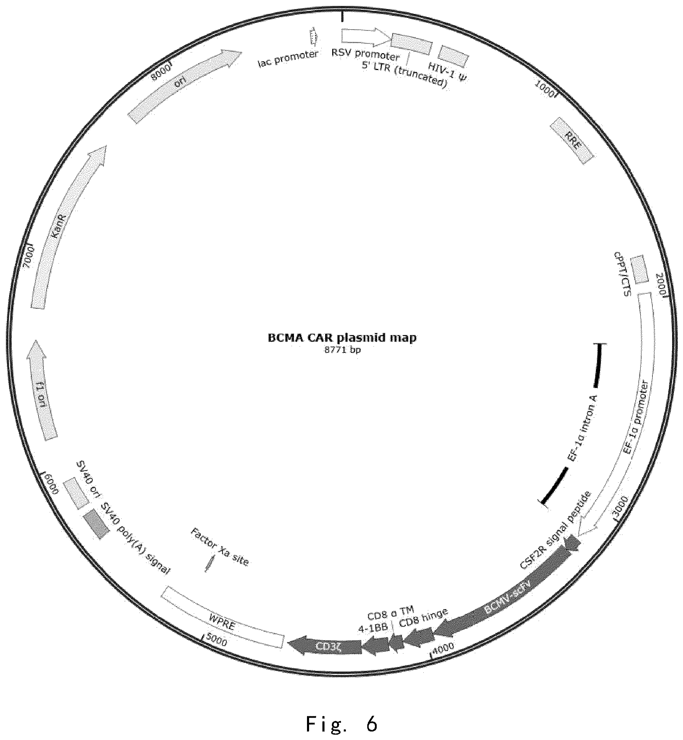

Anti-BCMA scFv antibody (Clone No. C11D5.3, SEQ ID NO: 2) and anti-CD19 scFv antibody (Clone No. FMC63, SEQ ID NO: 9) were selected, and fused with the corresponding hinge region, transmembrane region, co-stimulatory domain, and CD3ζ structural domain to form the BCMA CAR, the CD19-mutant CAR, and the CD19-wildtype CAR, respectively. The coding sequences of B19 CAR and B19-wildtype CAR were constructed by linking the coding sequences of CD19- and BCMA-monospecific CARs through a T2A sequence. The sequence synthesis and the preparation of lentiviral vector plasmids of B19 CAR, B19-wildtype CAR, CD19-mutant CAR, CD19-wildtype CAR and BCMA CAR were completed by Suzhou Genewiz Biotechnology Co., Ltd.
The structures expressing the above 5 CARs are shown in Fig. 1. The profiles of plasmids expressing the above 5 CARs are shown in Figs. 2-6.

**Example 2 Tumor cell culture and detection of cell surface antigen**

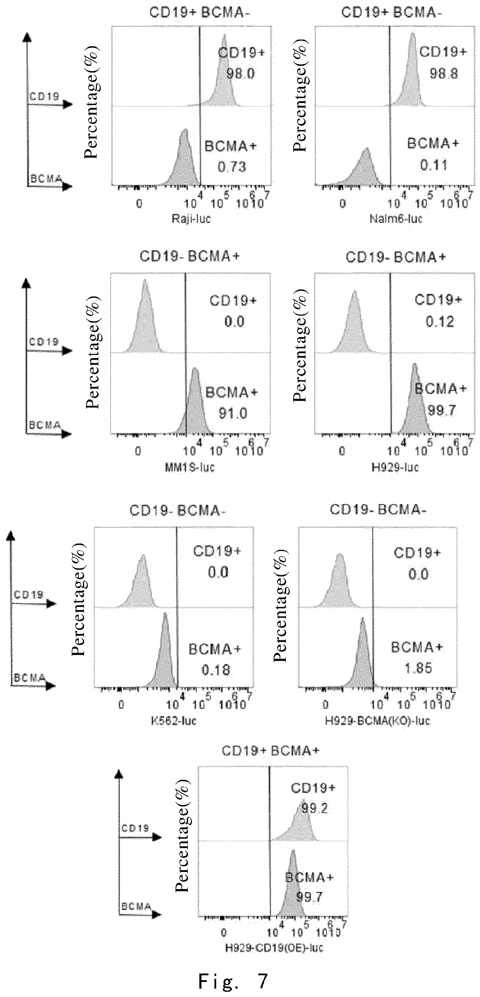

Tumor cells Nalm6-luc, Raji-luc, MM1S-luc, H929-luc, H929-CD19(OE)-luc, K562-luc, and H929-BCMA(KO)-luc expressing luciferase were thawed and transferred to culture flasks, and added with an appropriate amount of RPMI 1640+10% FBS+1% P/S. The culture flasks were then transferred to a cell culture incubator and cultured at 5% $CO_2$, 37°C, with cell density maintained at 0.5-2 M/mL.
Flow cytometry assay: 0.5-1 million tumor cells were taken into a 1.5 mL EP tube, and 1 mL of Rising buffer was added to resuspend the cells. The cells were centrifuged at 500 g for 5 minutes. The supernatant was discarded, and 100 μL of 1x Rising buffer was added to resuspend the cells. Antibodies were added to each tube, and incubated at 4°C in darkness for 30minutes. Then 1 mL of Rising buffer was added to wash the cells, followed by centrifugation at 500 g for 5minutes. The supernatant was discarded, and 200 μL of Rising buffer was added to resuspend the cells for analysis.
The results are shown in Fig. 7 and Table 1. The expressions of tumor cell surface antigens BCMA and CD19 are as follows: Raji-luc and Nalm6-luc are CD19+BCMA-; MM1S-luc and H929-luc are CD19-BCMA+; H929-CD19(OE)-luc is CD19+BCMA+; K562-luc and H929-BCMA(KO)-luc are CD19-BCMA-. All of the above 7 types of tumor cell express luciferase gene. H929-CD19(OE)-luc is a stably-transformed cell line overexpressing CD19 antigen, and H929-BCMA(KO)-luc is a stably-transformed cell line in which BCMA antigen has been knocked out.

**Table1 Expression of markers in tumor cells**

| Tumor cell | Expression of markers |
| --- | --- |
| Raji-luc | CD19+BCMA- |
| Nalm6-luc | CD19+BCMA- |
| MM1S-luc | CD19-BCMA+ |
| H929-luc | CD19-BCMA+ |
| H929-CD 19(OE)-luc | CD19+BCMA+ |
| K562-luc | CD19-BCMA- |
| H929-BCMA(KO)-luc | CD19-BCMA- |

**Example 3 Preparation of lentivirus**

The packaging of lentivirus was performed using PEI transfection reagent. 293TS cells were cultured in CD03 medium containing 6 mM L-glutamine until they reached optimal condition. A 4th-generation lentivirus packaging system was used. Two cell culture flasks were taken, and 5% of the total volume of suspension culture medium was added to the flasks. In Flask 1, CAR-expressing plasmids and packaging plasmids were added at a 4:2:2:1 ratio. In Flask 2, twice the total plasmid amount of PEI transfection reagent was added, mixed by shaking, and allowed to stand at room temperature for 5minutes. After the standing period, the PEI solution was transferred from Flask 2 to Flask 1 using a pipette, while gently shaking Flask 1. Once the solution was entirely added, the cap was put on, and the flask was gently shaken to mix. It was then allowed to stand at room temperature for 15 minutes for incubation. Afterwards, the PEI solution was transferred to a cell culture flask containing 293TS cells. The cell culture flask was then placed in a shaking $CO_2$ incubator and cultured at 37°C, 5% $CO_2$, and 140 r.p.m for another 48 hours.

The lentiviral supernatant in the cell culture flask was collected, and a hollow fiber column was used to remove small molecule impurities, such as HCP, nuclease and the like in the lentiviral supernatant. Capto Core 700 was used to separate protein particles of different objective sizes. A 0.22 $\mu$m bag filter was used to remove bacteria and other microbial impurities from the lentiviral supernatant. Finally, the lentiviral supernatant was concentrated to obtain a high-purity, high titer lentivirus.

## Example 4 Sorting and Activation of T Cells

After thawing the PBMCs, 9 volumes of Rinsing buffer were added for resuspension, followed by centrifugation at 300 g for 10minutes. The supernatant was discarded, and cells were resuspended at a certain ratio, with a corresponding volume of CD3 magnetic beads added. The mixture was gently mixed and incubated at 4°C for 30minutes, and mixed every 10 minutes. After being washed and resuspended, the cells were separated using MACS columns to obtain high purity CD3+ T cells.

The CD3+ T cells were diluted to 1.00E+6/mL using medium containing 10% FBS (X-VIVO-15 + 10% FBS + 1% P/S + 100 IU/mL IL-2). 2 mL of the cell suspension was placed into a 24-well plate, and 20 $\mu$L of TransACT activator was added to each well. The date was labeled as Day 0. The 24-well plate was then incubated in a 37°C $CO_2$ incubator for 48 hours, followed by centrifugation to remove the activator.

## Example 5 Transduction of T cell and CAR-T cell expansion

T cells activated for 48 hours were collected and resuspended in X-VIVO 15 + IL-2 to a concentration of 1 M/mL. 1 mL of T cells was added to each well of a 24-well plate, and lentiviruses for expressing B19 CAR, B19-wildtype CAR, CD19-mutant CAR, CD19-wildtype CAR, and BCMA CAR were added at an MOI of 3, respectively. A Mock T control well without virus was set up. 10 $\mu$L of DMSO was added to each well. The plate was incubated for 24 hours (Day 3) and added with fresh medium X-VIVO 15 + IL-2. On Day 4, cells were passaged by replacing the medium with complete culture medium (X-VIVO-15 + 10% FBS + 1% P/S + 100 U/mL IL-2). The CAR-T cell were passaged and cultured at a maintained density between 5.00E+5/mL~1.00E+6/mL. Every 2-3 days, CAR-T cells were collected, and cell counts and viability were assessed using a cell counter.

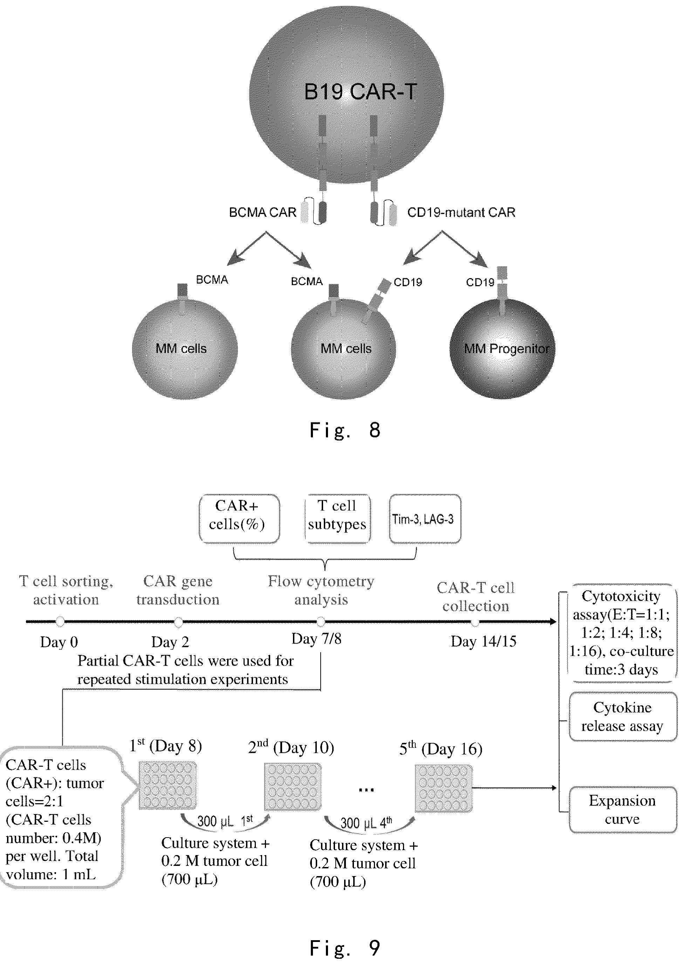

The schematic diagram of B19 CAR-T cells killing tumor cells is shown in Fig. 8. B19 CAR-T can kill CD19+ single-positive, BCMA+ single-positive, and CD19+BCMA+ double-positive tumor cells. The experimental workflow for CAR-T preparation is shown in Fig. 9.

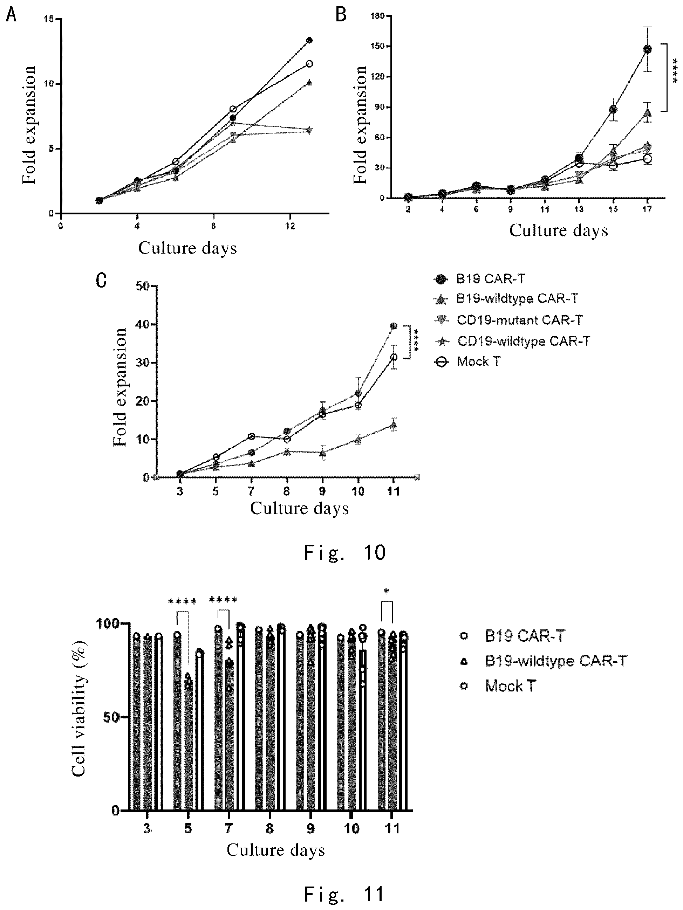

Unexpectedly, the cell expansion fold for cells comprising the CD28-YMFM mutant was significantly higher than that of CD28 wildtype (CD28-YMNM) CAR-T cells. As shown in Fig. 10(A), the expansion folds for B19 CAR-T, B19-wildtype CAR-T, CD19-mutant CAR-T, and CD19-wildtype CAR-T were 13.35, 10.12, 6.31, and 6.49, respectively. As shown in Fig. 10(B), the expansion folds for B19 CAR-T, B19-wildtype CAR-T, CD19-mutant CAR-T, and CD19-wildtype CAR-T were 147.38, 85.08, 47.66, 52.16, and 35.05, respectively. As shown in Fig. 10(C), the expansion folds for B19 CAR-T and B19-wildtype CAR-T were 40.19 and 13.02, respectively. These results indicate that B19 CAR-T exhibits better *in vitro* expansion compared with B19-wildtype CAR-T. Furthermore, it was unexpectedly found that the CD28-YMFM mutation also significantly improved the viability of CAR-T cells during the preparation phase. As shown in Fig. 11, during the CAR-T preparation phase, especially in the early stages, the viability of B19 CAR-T was 99.01%, which was much higher than the 78.5% viability of B19-wildtype CAR-T.

In summary, the mutation of the CD28 intracellular co-stimulatory domain improves the expansion fold and viability of B19 CAR-T, showing significant advantages.

## Example 6 Positivity rate, preparation success rate, phenotype and typing of CAR-T cells

CAR-T cells before (preparation phase) and after repeated stimulation were counted, and 0.5-1.00E+6 cells from each were placed into 1.5 mL EP tubes. Each tube was added with 1 mL of 1×Rising buffer, mixed, and centrifuged at 500 g for 5 min. The supernatant was discarded, and 100 $\mu$L of 1×Rising buffer was added to resuspend the cells. Commercially available detection antibodies were added to each tube according to the recommended amount, and the cells were incubated at 4°C in darkness for 30-40 minutes. Each tube was then washed with 1 mL of 1×Rising buffer, centrifuged at 500 g for 5 minutes, and the supernatant was discarded. Cells were resuspended in 300 $\mu$L of 1×Rising buffer, and flow cytometry analysis was performed.

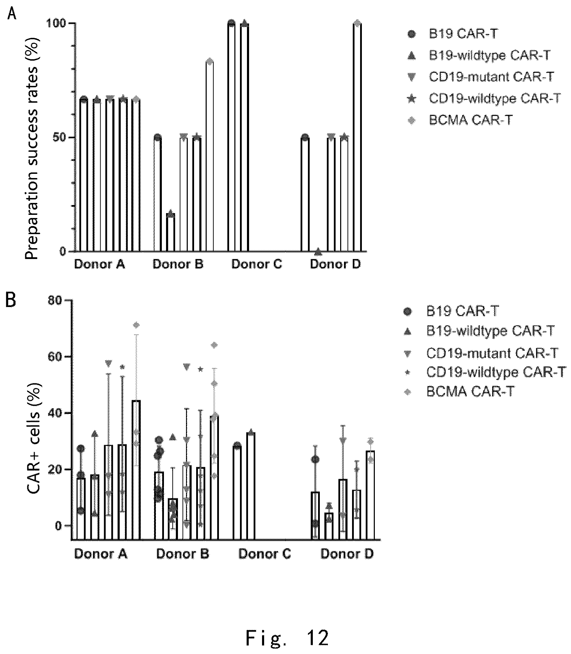

The results of this example are shown in Figs. 12-13. Fig. 12 shows that, by using B19 CAR lentivirus to transduce T cells from Donor B, six transductions were performed in total and three of them successfully prepared CAR-T cells. By using B19-wildtype CAR lentivirus to transduce T cells from Donor B, six transductions were performed in total and only one of them successfully prepared CAR-T cells. By using B19 CAR lentivirus to transduce T cells from Donor D, two transductions were performed in total and one of them successfully prepared CAR-T cells. By using B 19-wildtype CAR lentivirus to transduce T cells from Donor D, two transductions were performed in total and no CAR-T cell was successfully prepared.

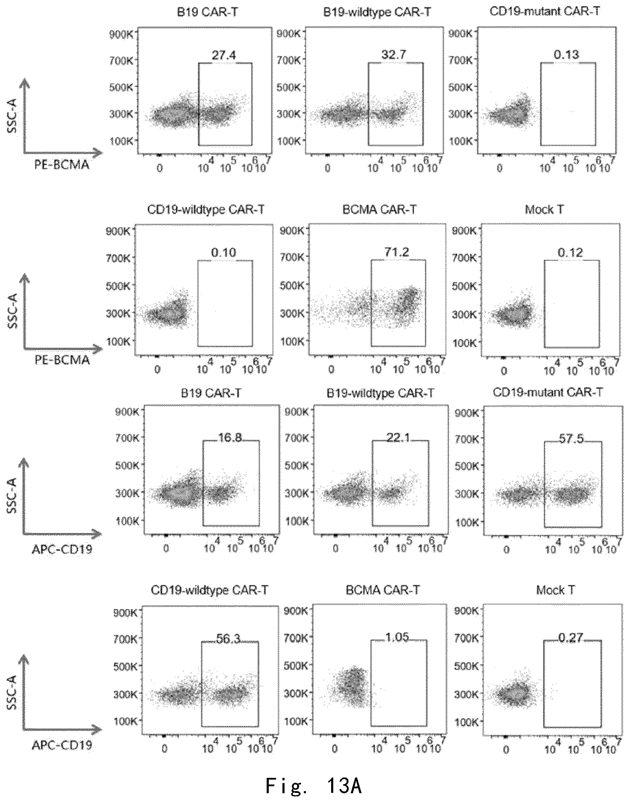

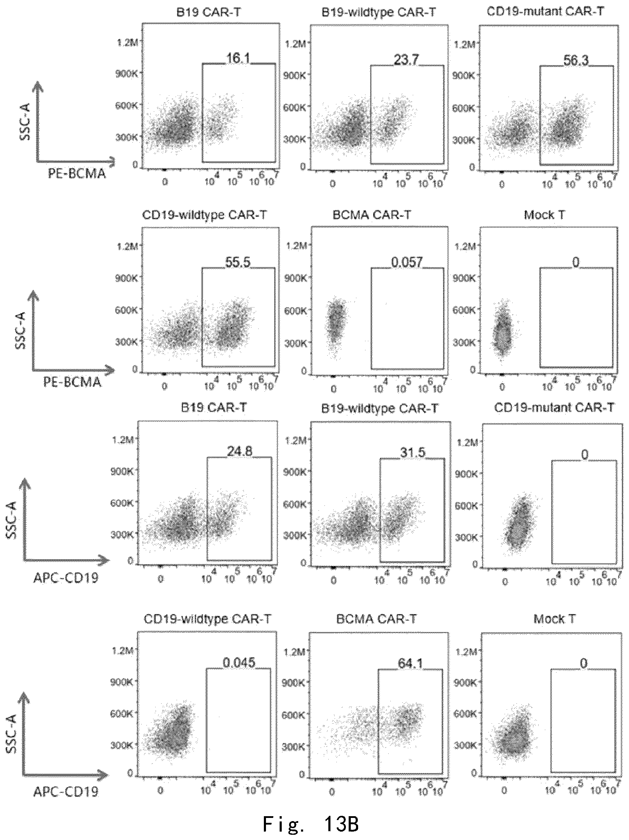

The preparation results of the four CAR-T groups show that, whether for multiple batches of cells from the same donor or for cells from different donor sources, the preparation success rate of B19 CAR-T was higher than that of B19-wildtype CAR-T, better meeting the urgent medication needs of patients and benefiting them. Since the difference between B19 CAR and B 19-wildtype CAR is a mutation in a single amino acid residue of the CD28 co-stimulatory domain, it is believed that the improvement in the preparation success rate of the CAR-T is likely due to the mutation in the single amino acid residue of CD28. Figs. 13A and 13B show that the successfully prepared CAR-T cells exhibited a positive rate >20%, which was used for subsequent experiments.

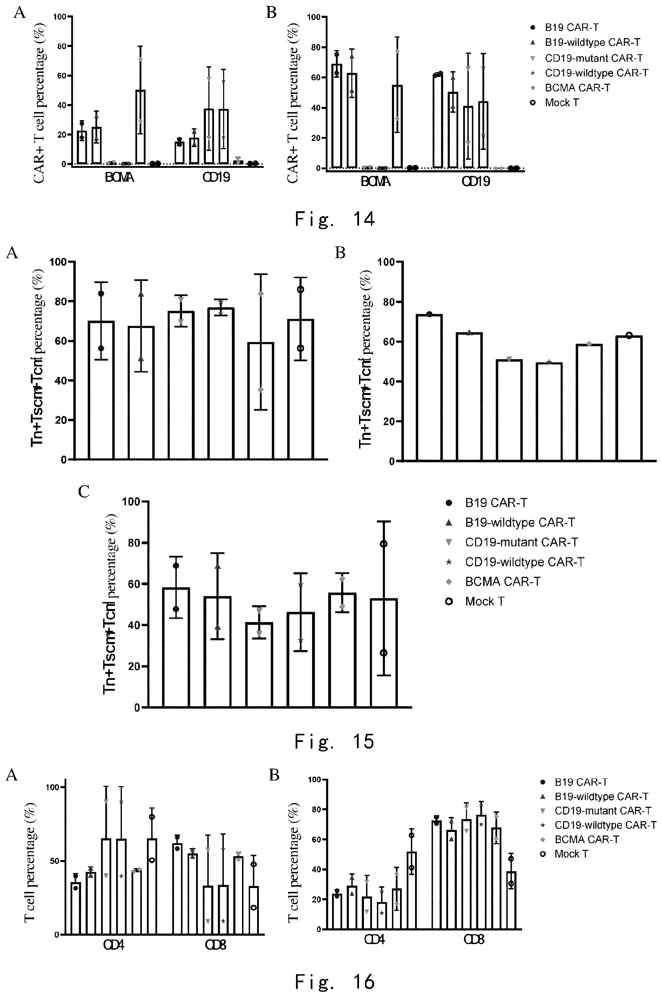

Fig. 14 shows that the proportion of CAR+ cells for B19 CAR-T, B19-wildtype CAR-T, CD19-mutant CAR-T, CD19-wildtype CAR-T, and BCMA CAR-T increased significantly after repeated stimulation.

Fig. 15 shows that before the repeated stimulation (i.e., during the preparation stage), no significant difference in the percentage of Tn+Tscm+Tcm cells in B19 CAR-T cells and the other four types of CAR-T cells was observed. However, after repeated stimulation with tumor cells, the percentage of Tn+Tscm+Tcm cells in B19 CAR-T was higher than that of the other four CAR-T types. This suggests that B19 CAR-T may have a better anti-tumor effect *in vivo.*

Fig. 16 shows that after repeated stimulation, the proportion of CD8+ T cells in B19 CAR-T, B19-wildtype CAR-T, CD19-mutant CAR-T, CD19-wildtype CAR-T, and BCMA CAR-T increased.

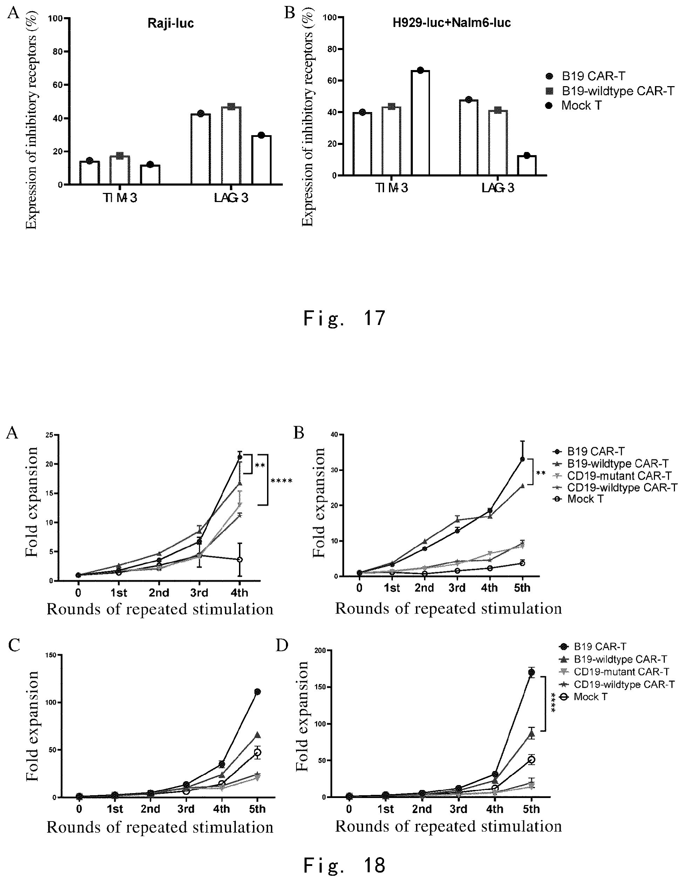

Fig. 17 shows that after three rounds of repeated stimulation with different tumor cells, there was no significant difference in the expression ratio of Tim-3 and LAG-3 between B19 CAR-T and B19-wildtype CAR-T.

In summary, the mutation in the CD28 intracellular co-stimulatory domain improves the preparation success rate of B19 CAR-T and the proportion of Tn+Tscm+Tcm cells after repeated stimulation.

**Example 7: Targeted Proliferation of CAR-T cells**

1. Preparation of target cells

Target cells in good growth condition were collected in 15 ml centrifuge tubes and centrifuged at 300 g for 5 min. The supernatant was discarded. The cells were resuspended in medium to a density of 1 M/ml and Mitomycin C was added at a final concentration of 1 $\mu$g/mL for treatment overnight. The next day, the cells were washed twice with 1$\times$Rising buffer, centrifuged at 300 g for 5 min, and the supernatant was discarded. The cells were then resuspended in X-VIVO-15 medium containing 10% FBS, counted using a Count star cell counter, and cell viability was assessed. The cell density was diluted to 1.00E+06/mL and reserved for further use.

2. Preparation of effector cells

B19 CAR-T, B19-wildtype CAR-T, CD19-mutant CAR-T, CD19-wildtype CAR-T, and Mock T cells were taken and centrifuged at 200 g for 10 min. The cells were resuspended in X-VIVO-15 medium and counted using a Count star cell counter. The cell density was adjusted to 1.00E+06/mL (based on the number of CAR-positive cells, with Mock T cells being based on the T cell count) and reserved for further use.

3. Co-incubation of effector and target cells

The prepared effector and target cells were added to a 24-well plate at an effector-to-target ratio of 2:1. 200 $\mu$L of target cells (0.20E+06/mL) and 400 $\mu$L of CAR-T cells (0.40E+06/mL) were added to each well. An additional 400 $\mu$L of X-VIVO-15 + 10% FBS + 1% P/S medium were added to each well. After adding the samples, the plate was gently shaken to ensure even mixing of the cells, which were then incubated at 37°C with 5% $CO_2$.

4. Multiple rounds of tumor cell stimulation and proliferation

Effector cells (CAR-T cells) and target cells (tumor cells) were co-incubated for 48 h. The cells in the 24-well plate were mixed using a pipette. The CAR-T cells were counted, and 300 $\mu$L of cells were transferred from the previous round to a plate for the next round. Each well was added with 200 $\mu$L of target cells (0.20E+06/mL). Then, 500 $\mu$L of X-VIVO-15 + 10% FBS + 1% P/S medium was added to each well to complete the culture system to 1 mL. The same procedure was repeated for the third, fourth, and fifth rounds of stimulation. Finally, the remaining tumor cells were observed and the final expansion fold of CAR-T cells was calculated.

The results are shown in Fig. 18. After repeated stimulation with tumor cells H929-luc, H929-luc+Nalm6-luc, and H929-CD19(OE)-luc, the expansion of B19 CAR-T cells was significantly superior to that of B19-wildtype CAR-T, CD19-mutant CAR-T and CD19-wildtype CAR-T, indicating that B19 CAR-T cells have a lasting ability to kill tumor cells.

**Example 8 Detection of killing activity of CAR-T cells before repeated stimulation**

1. Preparation of effector cells

B19 CAR-T, B19-wildtype CAR-T, CD19-mutant CAR-T, CD19-wildtype CAR-T, and Mock T cells were collected, centrifuged at 200 g for 10 minutes, and the supernatant was discarded. The cells were resuspended in X-VIVO 15 medium, counted using a Count Star cell counter, and the CAR-T cell density was adjusted to 2.00E+5 cells/mL, 1.00E+5 cells/mL, 5.00E+4 cells/mL, 2.50E+4 cells/mL, and 1.25E+4 cells/mL (calculated based on CAR-positive cells). The effector-to-target ratios were set to 1:1, 1:2, 1:4, 1:8 and 1:16.

2. Preparation of target cells

Healthy target cells were collected into a centrifuge tube and centrifuged at 400 g for 3 minutes, and the supernatant was discarded. The cells were resuspended in X-VIVO 15 medium and counted using a Count Star cell counter, and cell viability was checked. The final cell density was adjusted to 2.00E+5 cells/mL and reserved for further use.

3. Co-incubation of effector and target cells

100 $\mu$L of target cells and 100 $\mu$L of CAR-T cells were mixed at a ratio of 1:1 (with three replicates for each sample), added to a 96-well plate and incubated at 37°C, 5% $CO_2$ for 72 hours. After incubation, the samples were centrifuged at 500 g for 5 minutes, and 100 $\mu$L of the supernatant from each well was taken and stored in a -80°C freezer for subsequent cytokine detection. Then, 50 $\mu$L of ONE-GloTM luciferase Assay System detection substrate was added to each well. The mixture was gently shaken and left to stand for 5 minutes, followed by measurement of fluorescence using a TECAN plate reader at a wavelength of 560 nm.

4. Calculation of CAR-T cell killing rate.

$$\text{Killing rate (\%)} = (\text{Control group} - \text{Experimental group}) / \text{Control group} \times 100\%$$

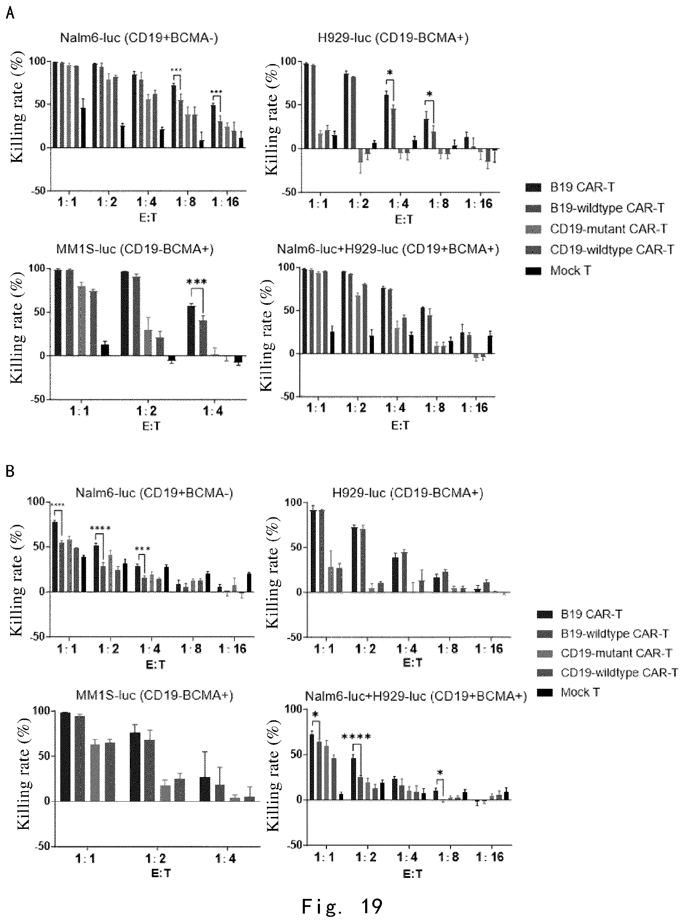

The results are shown in Fig. 19. For CD19+BCMA- cells (Nalm6-luc), the tumor cell killing rates of B19 CAR-T, B19-wildtype CAR-T, CD19-mutant CAR-T, and CD19-wildtype CAR-T were 71.80%, 54.99%, 38.38%, and 38.38%, respectively at lower E:T ratios, such as 1:8,. Similarly, the tumor cell killing rates of B19 CAR-T, B19-wildtype CAR-T, CD19-mutant CAR-T, and CD19-wildtype CAR-T were 49.04%, 30.61%, 24.60%, and 19.67%, respectively at an E:T ratio of 1:16. These results suggest that the B19 dual-target CAR-T cells have not only an expanded range of target cells but also a killing activity against target cells only expressing CD19 that is significantly higher than that of CD19 single-target CAR-T cells, especially at lower effector-to-target ratios.

The mutation in CD28 also affects the killing activity of CAR-T cells. At E:T ratios of 1:4 and 1:8, B19 CAR-T exhibited significantly better tumor cell killing activity against H929-luc compared with B19-wildtype CAR-T. At E:T ratios of 1:4, 1:8, and 1:16, B19 CAR-T showed significantly better tumor cell killing activity against MM1S-luc compared with B19-wildtype CAR-T.

In conclusion, the mutation in the intracellular co-stimulatory domain of CD28 enhances the tumor-killing effect of B19 CAR-T cells.

**Example 9: Detection of killing activity of CAR-T cells after repeated stimulation**

1. Preparation of effector cells

The B19 CAR-T, B19-wildtype CAR-T, CD19-mutant CAR-T, CD19-wildtype CAR-T, and Mock T cells were collected after repeated stimulation and centrifuged at 200 g for 10 minutes, and the supernatant was discarded. The cells were resuspended in X-VIVO 15 medium, counted using a Count Star cell counter, and the CAR-T cell density was adjusted to 2.00E+5 cells/mL, 1.00E+5 cells/mL, 5.00E+4 cells/mL, 2.50E+4 cells/mL, and 1.25E+4 cells/mL (calculated based on CAR-positive cells). The effector-to-target ratios were 1:1, 1:2, 1:4, 1:8, and 1:16. A control group without effector cells was set up.

2. Preparation of target cells

Healthy target cells were collected into a centrifuge tube and centrifuged at 400 g for 3 minutes, and the supernatant was discarded. The cells were resuspended in X-VIVO 15 medium and counted using a Count Star cell counter, and cell viability was checked. The final cell density was adjusted to 2.00E+5 cells/mL and reserved for further use.

3. Co-incubation of effector and target cells

100 $\mu$L of target cells and 100 $\mu$L of CAR-T cells were mixed at a ratio of 1:1 (with three replicates for each sample), added to a 96-well plate, with three replicates in total. The cells were co-incubated at 37°C with 5% $CO_2$ for 72 hours. After incubation, the samples were centrifuged at 500 g for 5 minutes, and 100 $\mu$L of the supernatant from each well was taken and stored in a -80°C freezer for subsequent cytokine detection. Then, 50 $\mu$L of ONE-GloTM luciferase Assay System detection substrate was added to each well. The mixture was gently shaken and left to stand for 5 minutes, followed by measurement of fluorescence using a TECAN plate reader at a wavelength of 560 nm.

5. Calculation of killing rate of CAR-T Cells

$$\text{Killing rate (\%)} = (\text{Control group} - \text{Experimental group}) / \text{Control group} \times 100\%$$

The results are shown in Fig. 19(B). After two rounds of repeated stimulation with tumor cells Nalm6-luc, the B19 CAR-T demonstrated significantly better killing effects against Nalm6-luc and Nalm6-luc + H929-luc cells compared with the B19-wildtype CAR-T.

These results indicate that, regardless of whether CAR-T cells were repeatedly stimulated with target cells, the B19 CAR-T exhibits significantly better tumor cell killing rate than B19-wildtype CAR-T and single-target CD19 CAR-T, demonstrating superior antitumor efficacy.

Example 8 together with Example 9 shows that regardless of whether CAR-T cells were repeatedly stimulated with target cells, the B19 CAR-T comprising a mutated CD28 intracellular co-stimulatory domain shows significantly better tumor-killing effects than those of B19 wildtype CAR-T and other single-target CAR-T cells.

**Example 10: Detection of cytokine release by CAR-T cell**

The supernatants before and after repeated stimulation were taken from the -80°C freezer for cytokine detection. An appropriate amount of 1.5 mL EP tubes were taken, and 50 $\mu$L of the respective co-cultured supernatant from each group was added to each tube. Centrifugation was performed at 500 g for 5 minutes, and 50 $\mu$L of the supernatant was collected for cytokine detection.

According to the instructions of the CBA detection kit, the cytokines to be detected and sample numbers were determined, and the capture beads were prepared. The magnetic beads bottle for cytokine capture was taken out and thoroughly mixed. For each type of bead, (sample number + negative control number) $\times$ 15 $\mu$L/6 of the capture beads was taken, and each type of bead was vortex-mixed. N 1.5 mL EP tubes were taken (n = sample number + control number), and 15 $\mu$L of the mixed beads was added to each tube (samples and negative controls). 15 $\mu$L of the corresponding reagent to be detected (sample or control) was added to each tube, followed by 15 $\mu$L of PE Detection Reagent. After thorough mixing, the tubes were incubated at room temperature in darkness for 3 hours. After incubation, 500 $\mu$L of wash buffer was added to each sample, mixed thoroughly, and centrifuged at 500 g for 5 minutes. The supernatant was discarded. The samples were resuspended in 100 $\mu$L of wash buffer, and centrifuged again at 500 g for 5 minutes, and the supernatant was discarded. The samples were then resuspended in 100 $\mu$L of wash buffer for flow cytometry detection.

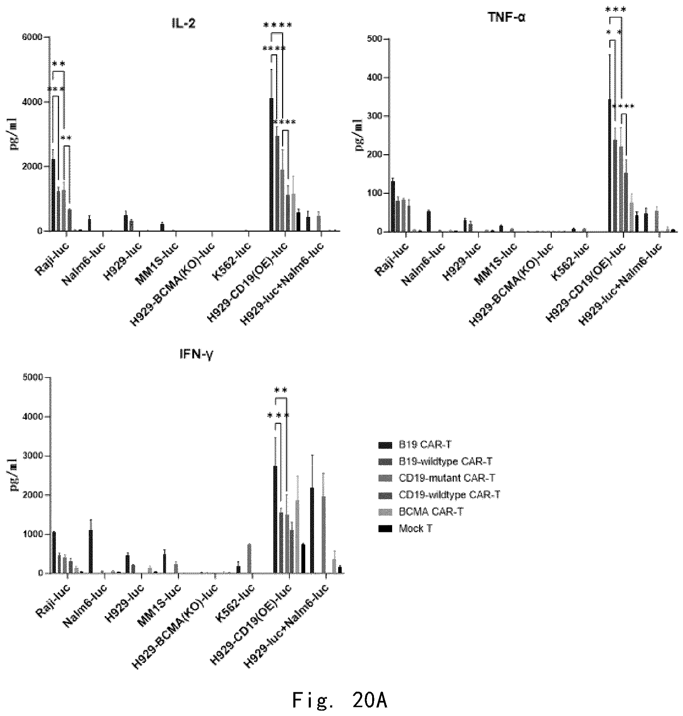

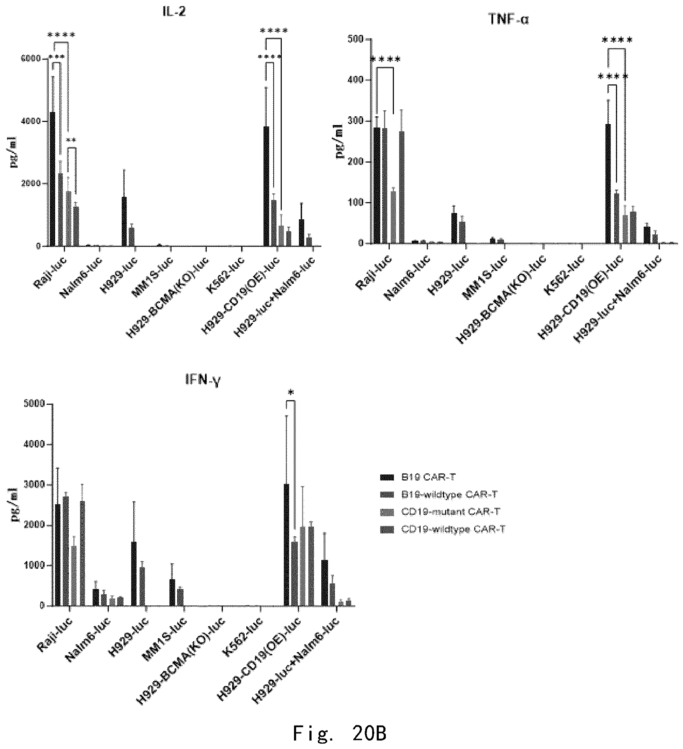

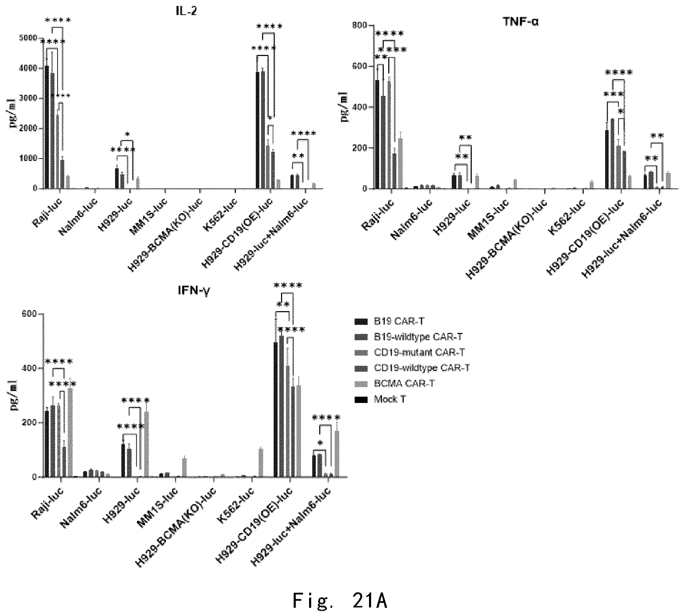

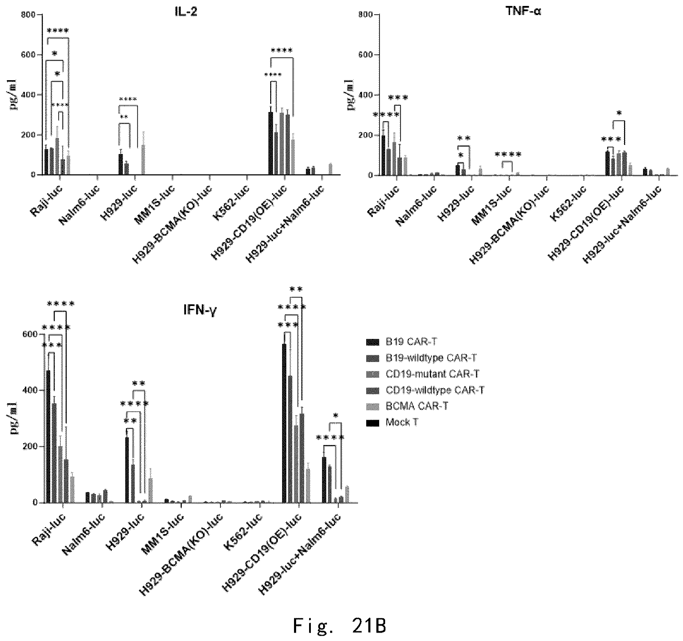

The results are shown in Figs. 20A, 20B, 21A, and 21B. The cytokine release from CAR-T cells co-cultured with tumor cells without repeated stimulation is shown in Fig. 20A (specific values are listed in Tables 2-4). After co-incubating CAR-T cells with tumor cells Raji-luc or H929-CD19(OE)-luc, the release of IL-2 from B19 CAR-T was significantly higher than that of B19-wildtype CAR-T, CD19-mutant CAR-T, CD19-wildtype CAR-T, and BCMA CAR-T. After co-incubating CAR-T cells with tumor cells H929-CD19(OE)-luc, the release of TNF-$\alpha$ from B19 CAR-T cells was significantly higher than that of B19-wildtype CAR-T, CD19-mutant CAR-T, CD19-wildtype CAR-T, and BCMA CAR-T cells. After co-incubating CAR-T cells with tumor cells H929-CD19(OE)-luc, the release of IFN-$\gamma$ from B19 CAR-T cells was significantly higher than that from B19-wildtype CAR-T and CD19-mutant CAR-T cells. This indicates that the single amino acid mutation in the co-stimulatory domain CD28 and the design of a parallel CAR structure can enhance cytokine release in B19 CAR-T cells, suggesting that B19 CAR-T with a parallel structure may have better therapeutic efficacy.

The cytokine release from CAR-T cells co-incubating with tumor cells after repeated stimulation with tumor cell Nalm6-luc is shown in Fig. 20B (specific values are listed in Tables 5-7). After co-incubating CAR-T cells repeatedly stimulated with tumor cells with tumor cells Raji-luc or H929-CD19(OE)-luc, the release of IL-2 from B19 CAR-T was significantly higher than that of B19-wildtype CAR-T and other single-target CAR-T cells, and the IL-2 release from CD19-mutant CAR-T was significantly higher than that from CD19-wildtype CAR-T. After co-incubating CAR-T cells repeatedly stimulated with tumor cells with tumor cells H929-CD19(OE)-luc, the release of IFN-$\gamma$ and TNF-$\alpha$ from B19 CAR-T cells was significantly higher than that from B19-wildtype CAR-T.

Fig. 21A-21B show similar results (specific values are shown in Tables 8-10 and Tables 11-13). Before and after repeated stimulation, the release of IL-2, TNF-$\alpha$, and IFN-$\gamma$ from B19 CAR-T cells upon co-incubating with target cells was significantly higher than that of B19-wildtype CAR-T and other single-target CAR-T cells, thus indicating that B19 CAR-T has superior expansion and tumor-killing effects *in vitro.*

In conclusion, regardless of whether CAR-T cells were repeatedly stimulated with tumor cells, the mutation in the intracellular co-stimulatory domain of CD28 significantly enhances the release of cytokines IL-2, TNF-$\alpha$, and IFN-$\gamma$ from B19 CAR-T cells.

**Table 2**

| Group | IL-2 (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19 CAR-T | Value 1 | 2540.96 | 512.28 | 653.57 | 282.08 | 2.88 | 5.1 | 5124.46 | 651.88 |
| | Value 2 | 1956.9 | 311.24 | 413.21 | 159.8 | 0.46 | 2.4 | 3814.53 | 358.33 |
| | Value 3 | 2170.88 | 309.52 | 406.74 | 202.55 | 1.82 | 2.58 | 3469.04 | 341.51 |
| | **Mean value** | **2222.91** | **377.68** | **491.17** | **214.81** | **1.72** | **3.36** | **4136.01** | **450.57** |
| B19-wildtype CAR-T | Value 1 | 1199.39 | 4.59 | 360.14 | 0 | 2.1 | 0 | 3056.69 | 0.63 |
| | Value 2 | 1127.06 | 4.21 | 308.44 | 0 | 0.58 | 0 | 3169.89 | 0 |
| | Value 3 | 1379.59 | 4.47 | 248.29 | 0 | 0.93 | 0 | 2626.99 | 0 |
| | **Mean value** | **1235.35** | **4.42** | **305.62** | **0.00** | **1.20** | **0.00** | **2951.19** | **0.21** |
| CD19-mutant CAR-T | Value 1 | 1065.09 | 0.97 | 0 | 19.1 | 2.41 | 14.24 | 1565.41 | 343.04 |
| | Value 2 | 1177.52 | 1.8 | 0.34 | 18.99 | 0.39 | 17.24 | 1542.67 | 395.57 |
| | Value 3 | 1547.62 | 0 | 1.42 | 30.23 | 2.27 | 26.28 | 2618.97 | 630.28 |
| | **Mean value** | **1263.41** | **0.92** | **0.59** | **22.77** | **1.69** | **19.25** | **1909.02** | **456.30** |
| CD19-wildtype CAR-T | Value 1 | 659.08 | 0 | 1.16 | 0 | 1.04 | 0 | 1025.51 | 0 |
| | Value 2 | 689.73 | 0 | 1.03 | 0 | 1.86 | 0 | 923.7 | 0 |
| | Value 3 | 692.57 | 0 | 2.03 | 0 | 3.08 | 0.58 | 1444.69 | 0.94 |
| | **Mean value** | **680.46** | **0.00** | **1.41** | **0.00** | **1.99** | **0.19** | **1131.30** | **0.31** |
| BCMA CAR-T | Value 1 | 64.24 | 21.95 | 34.95 | 0.51 | 12.46 | 0 | 1801.26 | 18.98 |
| | Value 2 | 38.06 | 21.52 | 21.91 | 0 | 2.5 | 0.42 | 823.29 | 19.34 |
| | Value 3 | 35.99 | 30.93 | 17.01 | 0 | 0.55 | 0.88 | 823.29 | 38.84 |
| | **Mean value** | **46.10** | **24.80** | **24.62** | **0.17** | **5.17** | **0.43** | **1149.28** | **25.72** |

(continued)

| Group | IL-2 (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| Mock T | Value 1 | 31.01 | 21.03 | 15.27 | 1.84 | 3.79 | 0 | 688.92 | 17.26 |
| | Value 2 | 35.23 | 19.34 | 15.83 | 1.83 | 3.91 | 0.31 | 581.57 | 20.15 |
| | Value 3 | 39.03 | 20.41 | 15.55 | 3.91 | 5.19 | 0 | 480.56 | 25 |
| | **Mean value** | **35.09** | **20.26** | **15.55** | **2.53** | **4.30** | **0.10** | **583.68** | **20.80** |

**Table 3**

| Group | TNF-α (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19 CAR-T | Value 1 | 135.58 | 54.74 | 36.19 | 18.32 | 0.83 | 10.23 | 474.24 | 63.48 |
| | Value 2 | 122.34 | 56.58 | 27.83 | 14.39 | 0.32 | 6.70 | 305.96 | 42.05 |
| | Value 3 | 137.98 | 52.06 | 24.92 | 16.01 | 0.82 | 5.51 | 254.55 | 38.97 |
| | **Mean value** | **131.97** | **54.46** | **29.65** | **16.24** | **0.66** | **7.48** | **344.92** | **48.17** |
| B19-wildtype CAR-T | Value 1 | 86.14 | 0.40 | 27.65 | 0.00 | 0.82 | 0.15 | 270.69 | 0.00 |
| | Value 2 | 70.35 | 0.52 | 20.98 | 0.00 | 0.51 | 0.00 | 235.69 | 0.00 |
| | Value 3 | 88.17 | 0.47 | 14.22 | 0.00 | 0.39 | 0.01 | 209.06 | 0.00 |
| | **Mean value** | **81.55** | **0.46** | **20.95** | **0.00** | **0.57** | **0.05** | **238.48** | **0.00** |
| CD19-mutant CAR-T | Value 1 | 77.90 | 3.41 | 0.00 | 8.35 | 0.22 | 9.31 | 193.36 | 44.54 |
| | Value 2 | 82.54 | 4.33 | 0.08 | 7.08 | 0.43 | 8.34 | 189.76 | 55.13 |
| | Value 3 | 86.40 | 3.30 | 0.00 | 9.11 | 0.49 | 6.56 | 279.28 | 65.90 |
| | **Mean value** | **82.28** | **3.68** | **0.03** | **8.18** | **0.38** | **8.07** | **220.80** | **55.19** |
| CD19-wildtype CAR-T | Value 1 | 84.56 | 0.00 | 0.11 | 0.00 | 0.48 | 0.00 | 138.58 | 0.00 |
| | Value 2 | 65.38 | 0.00 | 0.00 | 0.00 | 0.35 | 0.00 | 128.16 | 0.18 |
| | Value 3 | 54.35 | 0.03 | 0.16 | 0.00 | 0.69 | 0.00 | 191.94 | 0.41 |
| | **Mean value** | **68.10** | **0.01** | **0.09** | **0.00** | **0.51** | **0.00** | **152.89** | **0.20** |
| BCMA CAR-T | Value 1 | 6.95 | 3.23 | 5.67 | 0.11 | 0.88 | 0.02 | 102.32 | 5.69 |
| | Value 2 | 4.28 | 3.01 | 4.34 | 0.00 | 0.09 | 0.10 | 64.60 | 6.99 |
| | Value 3 | 4.60 | 5.00 | 4.13 | 0.00 | 0.07 | 0.00 | 59.37 | 13.73 |
| | **Mean value** | **5.28** | **3.75** | **4.71** | **0.04** | **0.35** | **0.04** | **75.43** | **8.80** |
| Mock T | Value 1 | 3.05 | 2.56 | 3.46 | 0.65 | 0.12 | 0.00 | 51.59 | 4.69 |
| | Value 2 | 3.08 | 2.59 | 3.05 | 0.69 | 0.67 | 0.00 | 45.36 | 4.59 |
| | Value 3 | 3.22 | 2.47 | 3.59 | 0.47 | 0.40 | 0.24 | 35.80 | 6.25 |
| | **Mean value** | **3.12** | **2.54** | **3.37** | **0.60** | **0.40** | **0.08** | **44.25** | **5.18** |

**Table 4**

| Group | IFN-γ (pg/ml) | Raji-luc | Nalm6-luc | H929-luc | MM1S-luc | H929-BCMA (KO)-luc | K562-luc | H929-CD19 (OE)-luc | H929-luc + Nalm6-luc |
|---|---|---|---|---|---|---|---|---|---|
| B19 CAR-T | Value 1 | 1064.74 | 1414.36 | 531.59 | 607.04 | 24.86 | 316.21 | 3548.98 | 3147.06 |
| | Value 2 | 1058.09 | 986.27 | 426.63 | 411.51 | 14.32 | 133.46 | 2518.62 | 1785.31 |
| | Value 3 | 1039.80 | 937.10 | 391.87 | 469.41 | 19.19 | 133.01 | 2139.01 | 1665.26 |
| | **Mean value** | **1054.21** | **1112.58** | **450.03** | **495.99** | **19.46** | **194.23** | **2735.54** | **2199.21** |

(continued)

| Group | IFN-γ (pg/ml) | Raji-luc | Nalm6-luc | H929-luc | MM1S-luc | H929-BCMA (KO)-luc | K562-luc | H929-CD19 (OE)-luc | H929-luc + Nalm6-luc |
|---|---|---|---|---|---|---|---|---|---|
| B19-wildtype CAR-T | Value 1 | 407.85 | 5.29 | 209.57 | 0.07 | 14.15 | 0.62 | 1504.47 | 2.57 |
| | Value 2 | 432.61 | 3.89 | 224.14 | 0.00 | 8.45 | 1.65 | 1667.76 | 0.73 |
| | Value 3 | 526.52 | 5.89 | 181.66 | 0.54 | 8.40 | 1.36 | 1447.31 | 1.45 |
| | **Mean value** | **455.66** | **5.02** | **205.12** | **0.20** | **10.33** | **1.21** | **1539.85** | **1.58** |
| CD19-mutant CAR-T | Value 1 | 345.98 | 41.43 | 0.86 | 200.35 | 6.31 | 758.55 | 1241.03 | 1553.00 |
| | Value 2 | 389.21 | 61.63 | 2.62 | 185.44 | 4.66 | 719.04 | 1195.80 | 1677.05 |
| | Value 3 | 483.66 | 68.17 | 1.52 | 310.29 | 8.40 | 694.28 | 2082.97 | 2649.54 |
| | **Mean value** | **406.28** | **57.08** | **1.67** | **232.03** | **6.46** | **723.96** | **1506.60** | **1959.86** |
| CD19-wildtype CAR-T | Value 1 | 385.35 | 0.00 | 1.73 | 0.00 | 4.09 | 0.00 | 1058.66 | 1.54 |
| | Value 2 | 312.02 | 0.56 | 1.24 | 0.00 | 4.07 | 1.51 | 953.80 | 0.97 |
| | Value 3 | 234.56 | 0.00 | 1.87 | 0.00 | 8.16 | 2.18 | 1325.75 | 2.08 |
| | **Mean value** | **310.64** | **0.19** | **1.61** | **0.00** | **5.44** | **1.23** | **1112.74** | **1.53** |
| BCMA CAR-T | Value 1 | 198.68 | 49.38 | 199.93 | 0.00 | 61.64 | 1.27 | 2579.30 | 232.13 |
| | Value 2 | 118.11 | 44.06 | 130.61 | 0.59 | 9.30 | 2.77 | 1566.63 | 267.04 |
| | Value 3 | 123.35 | 85.94 | 109.53 | 1.03 | 8.37 | 1.34 | 1495.52 | 606.60 |
| | **Mean value** | **146.71** | **59.79** | **146.69** | **0.54** | **26.44** | **1.79** | **1880.48** | **368.59** |
| Mock T | Value 1 | 31.93 | 38.22 | 45.83 | 3.34 | 6.60 | 2.19 | 754.38 | 151.70 |
| | Value 2 | 44.24 | 29.69 | 38.67 | 2.47 | 9.33 | 1.76 | 743.30 | 150.85 |
| | Value 3 | 46.52 | 27.95 | 44.66 | 2.15 | 14.78 | 1.82 | 699.05 | 200.02 |
| | **Mean value** | **40.90** | **31.95** | **43.05** | **2.65** | **10.24** | **1.92** | **732.24** | **167.52** |

**Table 5**

| Group | IL-2 (pg/ml) | Raji-luc | Nalm6-luc | H929-1uc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 5568.55 | 42.33 | 2577.92 | 77.38 | 0.00 | 3.87 | 5262.20 | 1452.87 |
| | Value 2 | 3906.04 | 18.11 | 1104.35 | 18.61 | 0.00 | 0.00 | 3260.08 | 625.49 |
| | Value 3 | 3336.02 | 15.86 | 1038.82 | 23.80 | 0.00 | 0.95 | 3039.49 | 546.25 |
| | **Mean value** | **4270.20** | **25.43** | **1573.70** | **39.93** | **0.00** | **1.61** | **3853.92** | **874.87** |
| B19-wild-type CAR-T | Value 1 | 2780.11 | 27.58 | 740.84 | 14.57 | 0.70 | 0.53 | 1668.90 | 407.07 |
| | Value 2 | 2117.27 | 22.69 | 505.71 | 14.70 | 0.52 | 0.73 | 1282.45 | 224.59 |
| | Value 3 | 2080.16 | 17.77 | 520.87 | 10.15 | 0.00 | 0.81 | 1482.15 | 209.38 |
| | **Mean value** | **2325.85** | **22.68** | **589.14** | **13.14** | **0.41** | **0.69** | **1477.83** | **280.35** |

(continued)

| Group | IL-2 (pg/ml) | Raji-luc | Nalm6-luc | H929-1 uc | MM1S-luc | H929-BCMA (KO)-luc | K562-luc | H929-CD19 (OE)-luc | H929-luc+ Nalm6-luc |
|---|---|---|---|---|---|---|---|---|---|
| CD19-mutant CAR-T | Value 1 | 1371.79 | 3.55 | 0.67 | 0.75 | 0.00 | 0.00 | 499.50 | 1.81 |
| | Value 2 | 1695.54 | 6.40 | 0.35 | 0.29 | 0.00 | 0.00 | 421.42 | 1.00 |
| | Value 3 | 2227.91 | 7.44 | 2.20 | 0.00 | 0.00 | 0.61 | 1063.99 | 5.11 |
| | **Mean value** | **1765.08** | **5.80** | **1.07** | **0.35** | **0.00** | **0.20** | **661.64** | **2.64** |
| CD19-wildtype CAR-T | Value 1 | 1113.39 | 3.33 | 0.00 | 0.77 | 0.00 | 0.26 | 377.76 | 0.61 |
| | Value 2 | 1342.42 | 6.56 | 0.00 | 0.00 | 0.00 | 0.00 | 410.10 | 0.00 |
| | Value 3 | 1345.91 | 7.18 | 0.79 | 0.00 | 0.00 | 0.51 | 629.48 | 1.54 |
| | **Mean value** | **1267.24** | **5.69** | **0.26** | **0.26** | **0.00** | **0.26** | **472.45** | **0.72** |

**Table 6**

| Group | TNF-α (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 311.71 | 7.37 | 95.80 | 15.49 | 0.34 | 0.58 | 358.95 | 52.19 |
| | Value 2 | 260.16 | 5.32 | 66.04 | 10.13 | 0.00 | 0.00 | 270.19 | 35.94 |
| | Value 3 | 279.76 | 6.06 | 61.71 | 10.72 | 0.12 | 0.02 | 251.74 | 32.97 |
| | **Mean value** | **283.88** | **6.25** | **74.52** | **12.11** | **0.15** | **0.20** | **293.63** | **40.37** |
| B19-wild-type CAR-T | Value 1 | 284.58 | 8.91 | 69.81 | 12.97 | 0.40 | 0.00 | 132.81 | 32.78 |
| | Value 2 | 237.71 | 5.60 | 45.07 | 9.27 | 0.00 | 0.47 | 116.40 | 19.53 |
| | Value 3 | 324.41 | 4.93 | 44.32 | 9.56 | 0.18 | 0.91 | 118.90 | 15.85 |
| | **Mean value** | **282.23** | **6.48** | **53.07** | **10.60** | **0.19** | **0.46** | **122.70** | **22.72** |
| CD19-mu-tant CAR-T | Value 1 | 121.42 | 3.88 | 0.05 | 0.00 | 0.00 | 0.05 | 62.39 | 2.33 |
| | Value 2 | 139.53 | 3.90 | 0.00 | 0.00 | 0.00 | 0.00 | 53.55 | 2.28 |
| | Value 3 | 121.33 | 4.48 | 0.17 | 0.00 | 0.02 | 0.00 | 97.03 | 3.17 |
| | **Mean value** | **127.43** | **4.09** | **0.07** | **0.00** | **0.01** | **0.02** | **70.99** | **2.59** |
| CD19-wild-type CAR-T | Value 1 | 300.51 | 2.46 | 0.00 | 0.24 | 0.38 | 0.00 | 62.00 | 1.68 |
| | Value 2 | 308.67 | 3.55 | 0.18 | 0.07 | 0.08 | 0.04 | 76.47 | 2.01 |
| | Value 3 | 210.16 | 4.13 | 0.33 | 0.00 | 0.34 | 0.04 | 91.78 | 3.26 |
| | **Mean value** | **273.11** | **3.38** | **0.17** | **0.10** | **0.27** | **0.03** | **76.75** | **2.32** |

**Table 7**

| Group | IFN-γ (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 3556.51 | 638.60 | 2733.65 | 1102.93 | 3.09 | 16.12 | 4964.01 | 1905.22 |
| | Value 2 | 2020.57 | 307.21 | 998.02 | 447.76 | 1.64 | 1.75 | 2045.65 | 820.95 |
| | Value 3 | 1956.23 | 293.93 | 1022.44 | 435.10 | 0.87 | 1.48 | 2078.26 | 686.71 |
| | **Mean value** | **2511.10** | **413.25** | **1584.70** | **661.93** | **1.87** | **6.45** | **3029.31** | **1137.63** |
| B19-wild-type CAR-T | Value 1 | 2744.24 | 404.53 | 1109.30 | 474.42 | 3.89 | 2.43 | 1734.27 | 777.18 |
| | Value 2 | 2559.17 | 222.94 | 793.28 | 382.05 | 1.91 | 4.42 | 1478.90 | 478.97 |
| | Value 3 | 2790.68 | 266.76 | 927.68 | 440.11 | 2.36 | 10.44 | 1549.40 | 447.64 |
| | **Mean value** | **2698.03** | **298.08** | **943.42** | **432.19** | **2.72** | **5.76** | **1587.52** | **567.93** |
| CD19-mutant CAR-T | Value 1 | 1311.55 | 120.05 | 1.75 | 2.11 | 0.26 | 1.58 | 1371.33 | 88.79 |
| | Value 2 | 1341.91 | 148.88 | 1.10 | 1.48 | 1.48 | 2.15 | 1430.47 | 70.16 |
| | Value 3 | 1764.00 | 264.11 | 6.43 | 1.70 | 1.40 | 2.50 | 3111.72 | 168.87 |
| | **Mean value** | **1472.49** | **177.68** | **3.09** | **1.76** | **1.05** | **2.08** | **1971.17** | **109.27** |
| CD19-wild-type CAR-T | Value 1 | 3034.27 | 192.41 | 1.62 | 3.92 | 2.56 | 0.36 | 1883.36 | 101.30 |
| | Value 2 | 2581.70 | 235.21 | 2.27 | 2.78 | 2.02 | 1.15 | 1879.94 | 91.82 |
| | Value 3 | 2207.22 | 220.40 | 5.48 | 3.34 | 1.41 | 1.19 | 2107.81 | 194.46 |
| | **Mean value** | **2607.73** | **216.01** | **3.12** | **3.35** | **2.00** | **0.90** | **1957.04** | **129.19** |

**Table 8**

| Group | IL-2 (pg/ml) | Raji-luc | Nalm6-luc | H929-1uc | MM1S-luc | H929-BCMA (KO)-luc | K562-luc | H929-CD19 (OE)-luc | H929-luc+ Nalm6-luc |
|---|---|---|---|---|---|---|---|---|---|
| B19 CAR-T | Value 1 | 4320.75 | 24.23 | 694.66 | 1.48 | 0.14 | 0.91 | 4090.17 | 448.06 |
| | Value 2 | 4083.05 | 24.06 | 558.25 | 1.51 | 0.00 | 0.55 | 3321.75 | 423.36 |
| | Value 3 | 3844.13 | 25.03 | 762.87 | 1.73 | 0.13 | 0.56 | 4224.73 | 447.05 |
| | **Mean value** | **4082.64** | **24.44** | **671.93** | **1.57** | **0.09** | **0.67** | **3878.88** | **439.49** |
| B19-wildtype CAR-T | Value 1 | 4534.47 | 41.98 | 507.67 | 2.18 | 0.00 | 2.16 | 3865.64 | 410.33 |
| | Value 2 | 3873.60 | 39.66 | 528.57 | 3.28 | 0.20 | 4.37 | 3824.13 | 445.93 |
| | Value 3 | 3118.79 | 40.97 | 405.19 | 3.11 | 0.50 | 4.85 | 4020.24 | 474.00 |
| | **Mean value** | **3842.29** | **40.87** | **480.48** | **2.86** | **0.23** | **3.79** | **3903.34** | **443.42** |
| CD19-mutant CAR-T | Value 1 | 2635.96 | 11.40 | 0.08 | 0.11 | 0.04 | 0.00 | 1183.48 | 0.90 |
| | Value 2 | 2417.91 | 14.64 | 0.22 | 0.00 | 0.16 | 0.39 | 1533.41 | 1.38 |
| | Value 3 | 2360.96 | 9.91 | 0.00 | 0.06 | 0.00 | 0.05 | 1541.64 | 1.49 |
| | **Mean value** | **2471.61** | **11.98** | **0.10** | **0.06** | **0.07** | **0.15** | **1419.51** | **1.26** |

(continued)

| Group | IL-2 (pg/ml) | Raji-luc | Nalm6-luc | H929-1 uc | MM1S-luc | H929-BCMA (KO)-luc | K562-luc | H929-CD19 (OE)-luc | H929-luc+ Nalm6-luc |
|---|---|---|---|---|---|---|---|---|---|
| CD19-wildtype CAR-T | Value 1 | 1088.32 | 16.08 | 0.60 | 0.67 | 0.00 | 1.80 | 1317.90 | 1.97 |
| | Value 2 | 894.14 | 18.26 | 0.90 | 0.86 | 0.05 | 1.38 | 1168.58 | 2.18 |
| | Value 3 | 863.50 | 16.25 | 0.35 | 0.60 | 0.11 | 0.87 | 1175.80 | 1.48 |
| | **Mean value** | **948.65** | **16.86** | **0.62** | **0.71** | **0.05** | **1.35** | **1220.76** | **1.88** |
| BCMA CAR-T | Value 1 | 449.61 | 0.18 | 372.27 | 3.36 | 0.36 | 9.58 | 279.50 | 182.56 |
| | Value 2 | 390.07 | 0.07 | 295.61 | 2.82 | 0.00 | 8.88 | 264.71 | 129.08 |
| | Value 3 | 349.41 | 0.42 | 363.84 | 3.15 | 0.47 | 7.17 | 302.81 | 177.88 |
| | **Mean value** | **396.36** | **0.22** | **343.91** | **3.11** | **0.28** | **8.54** | **282.34** | **163.17** |
| Mock T | Value 1 | 8.49 | 0.00 | 0.00 | 0.00 | 0.00 | 0.00 | 0.08 | 0.00 |
| | Value 2 | 4.61 | 0.00 | 0.00 | 0.00 | 0.02 | 0.00 | 0.00 | 0.00 |
| | Value 3 | 9.48 | 0.00 | 0.00 | 0.01 | 0.00 | 0.03 | 0.00 | 0.00 |
| | **Mean value** | **7.53** | **0.00** | **0.00** | **0.00** | **0.01** | **0.01** | **0.03** | **0.00** |

**Table 9**

| Group | TNF-α (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 578.47 | 11.22 | 68.78 | 7.80 | 0.60 | 1.16 | 291.20 | 62.19 |
| | Value 2 | 546.63 | 10.60 | 55.42 | 9.98 | 0.44 | 1.25 | 253.09 | 64.39 |
| | Value 3 | 471.83 | 9.80 | 75.56 | 9.64 | 0.00 | 0.95 | 323.44 | 71.25 |
| | **Mean value** | **532.31** | **10.54** | **66.59** | **9.14** | **0.35** | **1.12** | **289.24** | **65.94** |
| B19-wild-type CAR-T | Value 1 | 532.27 | 16.82 | 69.00 | 14.47 | 1.25 | 2.20 | 337.25 | 79.90 |
| | Value 2 | 458.06 | 18.04 | 76.33 | 19.32 | 0.80 | 5.25 | 338.14 | 85.00 |
| | Value 3 | 378.79 | 19.89 | 49.07 | 16.17 | 1.88 | 5.70 | 343.40 | 86.70 |
| | **Mean value** | **456.37** | **18.25** | **64.80** | **16.65** | **1.31** | **4.38** | **339.60** | **83.87** |
| CD19-mutant CAR-T | Value 1 | 547.74 | 17.00 | 0.16 | 0.95 | 0.22 | 0.66 | 183.36 | 8.12 |
| | Value 2 | 525.39 | 19.19 | 0.63 | 0.94 | 0.61 | 1.33 | 240.96 | 9.79 |
| | Value 3 | 504.94 | 13.07 | 0.28 | 0.59 | 0.53 | 0.75 | 213.90 | 9.11 |
| | **Mean value** | **526.02** | **16.42** | **0.36** | **0.83** | **0.45** | **0.91** | **212.74** | **9.01** |
| CD19-wildtype CAR-T | Value 1 | 199.20 | 15.01 | 0.74 | 4.11 | 0.90 | 3.04 | 186.15 | 9.60 |
| | Value 2 | 171.04 | 17.23 | 0.99 | 3.43 | 0.96 | 3.42 | 181.57 | 12.02 |
| | Value 3 | 142.37 | 17.43 | 1.06 | 2.89 | 0.85 | 3.48 | 185.51 | 8.27 |
| | **Mean value** | **170.87** | **16.56** | **0.93** | **3.48** | **0.90** | **3.31** | **184.41** | **9.96** |
| BCMA CAR-T | Value 1 | 282.17 | 7.91 | 66.77 | 46.36 | 4.04 | 42.08 | 60.30 | 86.47 |
| | Value 2 | 244.67 | 6.14 | 52.67 | 41.73 | 2.42 | 34.30 | 54.56 | 65.29 |
| | Value 3 | 219.48 | 6.46 | 68.57 | 36.37 | 5.46 | 32.71 | 66.47 | 78.02 |
| | **Mean value** | **248.77** | **6.84** | **62.67** | **41.49** | **3.97** | **36.36** | **60.44** | **76.59** |

(continued)

| Group | TNF-α (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| Mock T | Value 1 | 3.95 | 0.70 | 0.00 | 0.08 | 0.21 | 0.02 | 0.09 | 0.00 |
| | Value 2 | 2.25 | 0.43 | 0.00 | 0.43 | 0.00 | 0.00 | 0.00 | 0.19 |
| | Value 3 | 4.00 | 0.96 | 0.00 | 0.00 | 0.25 | 0.52 | 0.02 | 0.06 |
| | **Mean value** | **3.40** | **0.70** | **0.00** | **0.17** | **0.15** | **0.18** | **0.04** | **0.08** |

**Table 10**

| Group | INF-γ (pg/ml) | Raji-luc | Nalm6-luc | H929-1 uc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 240.53 | 21.00 | 116.07 | 11.10 | 1.47 | 1.59 | 474.11 | 77.85 |
| | Value 2 | 256.94 | 20.29 | 103.29 | 13.11 | 1.59 | 1.16 | 427.02 | 80.00 |
| | Value 3 | 231.96 | 19.53 | 137.62 | 13.37 | 1.22 | 1.14 | 591.31 | 78.47 |
| | **Mean value** | **243.14** | **20.27** | **118.99** | **12.53** | **1.43** | **1.30** | **497.48** | **78.77** |
| B19-wild-type CAR-T | Value 1 | 284.86 | 22.35 | 104.86 | 14.29 | 2.42 | 3.05 | 486.27 | 82.55 |
| | Value 2 | 280.46 | 28.00 | 121.82 | 15.82 | 2.15 | 6.37 | 532.78 | 84.15 |
| | Value 3 | 227.04 | 29.88 | 87.09 | 15.39 | 2.57 | 6.50 | 542.06 | 86.11 |
| | **Mean value** | **264.12** | **26.74** | **104.59** | **15.17** | **2.38** | **5.31** | **520.37** | **84.27** |
| CD19-mutant CAR-T | Value 1 | 261.72 | 19.86 | 1.14 | 1.59 | 0.87 | 0.60 | 339.90 | 12.23 |
| | Value 2 | 260.98 | 25.79 | 2.00 | 1.61 | 1.34 | 1.16 | 446.77 | 14.15 |
| | Value 3 | 272.76 | 19.81 | 1.30 | 2.03 | 1.03 | 0.99 | 448.42 | 12.59 |
| | **Mean value** | **265.15** | **21.82** | **1.48** | **1.74** | **1.08** | **0.92** | **411.70** | **12.99** |
| CD19-wildtype CAR-T | Value 1 | 138.19 | 17.20 | 1.82 | 4.69 | 2.28 | 4.44 | 359.98 | 13.87 |
| | Value 2 | 92.36 | 19.36 | 2.95 | 4.42 | 1.77 | 4.40 | 340.28 | 13.13 |
| | Value 3 | 100.02 | 20.11 | 2.36 | 3.72 | 1.81 | 4.08 | 302.68 | 10.62 |
| | **Mean value** | **110.19** | **18.89** | **2.38** | **4.28** | **1.95** | **4.31** | **334.31** | **12.54** |
| BCMA CAR-T | Value 1 | 342.61 | 13.52 | 261.19 | 79.61 | 11.47 | 112.02 | 345.86 | 202.62 |
| | Value 2 | 353.44 | 11.26 | 198.24 | 67.76 | 7.84 | 96.56 | 299.23 | 138.51 |
| | Value 3 | 280.13 | 12.13 | 258.99 | 58.75 | 12.32 | 100.63 | 363.26 | 171.48 |
| | **Mean value** | **325.39** | **12.30** | **239.47** | **68.71** | **10.54** | **103.07** | **336.12** | **170.87** |
| Mock T | Value 1 | 1.74 | 0.17 | 0.38 | 0.00 | 0.00 | 0.31 | 0.13 | 0.00 |
| | Value 2 | 1.02 | 0.00 | 0.00 | 0.00 | 0.00 | 0.00 | 0.00 | 0.05 |
| | Value 3 | 2.31 | 0.00 | 0.34 | 0.31 | 0.10 | 0.00 | 0.00 | 0.00 |
| | **Mean value** | **1.69** | **0.06** | **0.24** | **0.10** | **0.03** | **0.10** | **0.04** | **0.02** |

**Table 11**

| Group | IL-2 (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 104.12 | 0.00 | 96.83 | 0.00 | 0.00 | 0.00 | 318.45 | 37.59 |
| | Value 2 | 132.05 | 0.02 | 87.58 | 0.14 | 0.00 | 0.00 | 339.23 | 19.07 |
| | Value 3 | 147.32 | 0.00 | 131.23 | 0.00 | 0.00 | 0.00 | 288.34 | 31.58 |
| | **Mean value** | **127.83** | **0.01** | **105.21** | **0.05** | **0.00** | **0.00** | **315.34** | **29.41** |
| B19-wild-type CAR-T | Value 1 | 135.15 | 0.06 | 60.21 | 0.10 | 0.00 | 0.00 | 186.13 | 34.83 |
| | Value 2 | 129.61 | 0.16 | 48.83 | 0.00 | 0.20 | 0.00 | 192.64 | 29.09 |
| | Value 3 | 133.70 | 0.56 | 66.90 | 0.02 | 0.00 | 0.00 | 258.38 | 42.60 |
| | **Mean value** | **132.82** | **0.26** | **58.65** | **0.04** | **0.07** | **0.00** | **212.38** | **35.51** |
| CD19-mutant CAR-T | Value 1 | 234.00 | 1.45 | 0.00 | 0.08 | 0.00 | 0.00 | 335.12 | 0.00 |
| | Value 2 | 199.50 | 0.52 | 0.00 | 0.00 | 0.00 | 0.00 | 294.92 | 0.02 |
| | Value 3 | 116.21 | 0.73 | 0.00 | 0.00 | 0.12 | 0.28 | 307.92 | 0.00 |
| | **Mean value** | **183.24** | **0.90** | **0.00** | **0.03** | **0.04** | **0.09** | **312.65** | **0.01** |
| CD19-wildtype CAR-T | Value 1 | 152.01 | 0.60 | 0.00 | 0.11 | 0.32 | 0.00 | 292.90 | 0.00 |
| | Value 2 | 49.16 | 0.71 | 0.00 | 0.00 | 0.35 | 0.00 | 328.35 | 0.00 |
| | Value 3 | 29.08 | 0.52 | 0.27 | 0.00 | 0.00 | 0.00 | 284.06 | 0.00 |
| | **Mean value** | **76.75** | **0.61** | **0.09** | **0.04** | **0.22** | **0.00** | **301.77** | **0.00** |
| BCMA CAR-T | Value 1 | 115.76 | 0.29 | 224.90 | 1.72 | 0.04 | 0.39 | 211.36 | 56.56 |
| | Value 2 | 68.94 | 0.26 | 120.15 | 2.57 | 0.10 | 0.11 | 153.95 | 49.29 |
| | Value 3 | 101.92 | 0.14 | 108.12 | 1.99 | 0.29 | 0.00 | 162.17 | 54.38 |
| | **Mean value** | **95.54** | **0.23** | **151.06** | **2.09** | **0.14** | **0.17** | **175.83** | **53.41** |
| Mock T | Value 1 | 0.00 | 0.04 | 0.00 | 0.00 | 0.00 | 0.00 | 0.00 | 0.00 |
| | Value 2 | 0.00 | 0.00 | 0.00 | 0.00 | 0.00 | 0.00 | 0.00 | 0.00 |
| | Value 3 | 0.00 | 0.16 | 0.00 | 0.00 | 0.00 | 0.00 | 0.00 | 0.00 |
| | **Mean value** | **0.00** | **0.07** | **0.00** | **0.00** | **0.00** | **0.00** | **0.00** | **0.00** |

**Table 12**

| Group | TNF-$\alpha$ (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| B19CAR-T | Value 1 | 164.22 | 3.72 | 44.50 | 1.26 | 0.76 | 0.13 | 111.99 | 36.96 |
| | Value 2 | 217.14 | 3.21 | 48.15 | 1.37 | 0.66 | 0.00 | 122.26 | 24.83 |
| | Value 3 | 211.17 | 3.90 | 57.38 | 1.74 | 0.39 | 0.26 | 120.05 | 36.64 |
| | **Mean value** | **197.51** | **3.61** | **50.01** | **1.46** | **0.60** | **0.13** | **118.10** | **32.81** |
| B19-wild-type CAR-T | Value 1 | 126.36 | 3.39 | 30.80 | 1.06 | 0.13 | 0.00 | 78.08 | 28.04 |
| | Value 2 | 129.51 | 3.36 | 26.01 | 0.71 | 0.00 | 0.00 | 77.22 | 22.93 |
| | Value 3 | 132.29 | 4.04 | 27.22 | 0.82 | 0.30 | 0.05 | 98.49 | 27.16 |
| | **Mean value** | **129.39** | **3.60** | **28.01** | **0.86** | **0.14** | **0.02** | **84.60** | **26.04** |
| CD19-mutant CAR-T | Value 1 | 192.08 | 11.99 | 0.95 | 0.05 | 0.68 | 0.75 | 124.49 | 3.48 |
| | Value 2 | 194.30 | 7.63 | 1.10 | 0.79 | 0.70 | 0.26 | 107.06 | 4.09 |
| | Value 3 | 111.37 | 7.74 | 0.57 | 0.68 | 0.51 | 0.06 | 102.02 | 3.86 |
| | **Mean value** | **165.92** | **9.12** | **0.87** | **0.51** | **0.63** | **0.36** | **111.19** | **3.81** |

(continued)

| Group | TNF-α (pg/ml) | Raji-luc | Nalm6-luc | H929-luc | MM1S-luc | H929-BCMA (KO)-luc | K562-luc | H929-CD19 (OE)-luc | H929-luc+ Nalm6-luc |
|---|---|---|---|---|---|---|---|---|---|
| CD19- wildtype CAR-T | Value 1 | 162.79 | 12.44 | 0.94 | 1.27 | 1.10 | 0.56 | 110.28 | 4.07 |
| | Value 2 | 68.66 | 11.66 | 0.42 | 0.90 | 0.78 | 0.70 | 116.72 | 2.93 |
| | Value 3 | 37.79 | 13.86 | 0.34 | 0.91 | 0.53 | 0.00 | 121.15 | 4.12 |
| | **Mean value** | **89.75** | **12.65** | **0.57** | **1.03** | **0.80** | **0.42** | **116.05** | **3.71** |
| BCMA CAR-T | Value 1 | 100.77 | 2.70 | 48.99 | 11.74 | 2.02 | 0.39 | 62.06 | 36.26 |
| | Value 2 | 73.71 | 2.36 | 30.74 | 12.98 | 1.21 | 0.73 | 48.28 | 31.13 |
| | Value 3 | 86.30 | 2.69 | 27.83 | 14.58 | 1.80 | 1.22 | 45.76 | 34.04 |
| | **Mean value** | **86.93** | **2.58** | **35.85** | **13.10** | **1.68** | **0.78** | **52.03** | **33.81** |
| Mock T | Value 1 | 0.63 | 0.01 | 0.00 | 0.19 | 0.32 | 0.27 | 0.00 | 0.22 |
| | Value 2 | 0.17 | 0.27 | 0.21 | 0.02 | 0.43 | 0.35 | 0.48 | 0.00 |
| | Value 3 | 0.58 | 0.44 | 0.00 | 0.05 | 0.41 | 0.11 | 0.11 | 0.14 |
| | **Mean value** | **0.46** | **0.24** | **0.07** | **0.09** | **0.39** | **0.24** | **0.20** | **0.12** |

**Table 13**

| Group | INF-γ (pg/ml) | Raji-luc | Nalm6-luc | H929-luc | MM1S-luc | H929-BCMA (KO)-luc | K562-1 uc | H929-CD19 (OE)-luc | H929-luc+ Nalm6-luc |
|---|---|---|---|---|---|---|---|---|---|
| B19 CAR-T | Value 1 | 420.44 | 35.71 | 208.25 | 11.89 | 3.29 | 3.28 | 526.43 | 174.40 |
| | Value 2 | 530.81 | 36.83 | 239.47 | 12.27 | 2.95 | 3.24 | 598.98 | 141.13 |
| | Value 3 | 463.42 | 35.32 | 251.00 | 13.83 | 3.32 | 3.16 | 570.16 | 168.99 |
| | **Mean value** | **471.56** | **35.95** | **232.91** | **12.66** | **3.19** | **3.23** | **565.19** | **161.51** |
| B19- wildtype CAR-T | Value 1 | 320.73 | 31.82 | 121.12 | 7.24 | 2.03 | 2.67 | 379.28 | 127.49 |
| | Value 2 | 365.98 | 31.34 | 126.67 | 7.02 | 2.33 | 1.91 | 426.97 | 123.39 |
| | Value 3 | 370.99 | 30.25 | 155.30 | 7.00 | 2.53 | 2.26 | 555.83 | 135.04 |
| | **Mean value** | **352.57** | **31.14** | **134.36** | **7.09** | **2.30** | **2.28** | **454.03** | **128.64** |
| CD19- mutant CAR-T | Value 1 | 219.01 | 33.26 | 6.29 | 0.63 | 2.77 | 4.55 | 315.66 | 15.65 |
| | Value 2 | 226.71 | 20.24 | 5.86 | 3.91 | 2.78 | 4.98 | 251.76 | 14.57 |
| | Value 3 | 162.19 | 23.20 | 5.26 | 3.28 | 1.54 | 4.68 | 259.72 | 16.13 |
| | **Mean value** | **202.64** | **25.57** | **5.80** | **2.61** | **2.36** | **4.74** | **275.71** | **15.45** |
| CD19- wildtype CAR-T | Value 1 | 287.43 | 45.90 | 8.45 | 8.32 | 8.34 | 7.00 | 296.44 | 22.21 |
| | Value 2 | 106.77 | 41.30 | 7.52 | 8.56 | 7.45 | 6.52 | 342.63 | 17.36 |
| | Value 3 | 68.30 | 47.51 | 4.97 | 8.05 | 5.76 | 4.89 | 313.80 | 21.58 |
| | **Mean value** | **154.17** | **44.90** | **6.98** | **8.31** | **7.18** | **6.14** | **317.62** | **20.38** |
| BCMA CAR-T | Value 1 | 104.18 | 6.41 | 129.25 | 22.35 | 5.74 | 0.39 | 143.53 | 62.26 |
| | Value 2 | 77.14 | 5.08 | 64.61 | 22.80 | 5.71 | 5.76 | 100.62 | 53.92 |
| | Value 3 | 97.92 | 5.94 | 67.38 | 25.25 | 5.45 | 5.52 | 115.18 | 54.80 |
| | **Mean value** | **93.08** | **5.81** | **87.08** | **23.47** | **5.63** | **3.89** | **119.78** | **56.99** |
| Mock T | Value 1 | 0.00 | 0.12 | 0.00 | 0.39 | 0.00 | 0.00 | 0.02 | 0.13 |
| | Value 2 | 0.00 | 0.00 | 0.08 | 0.00 | 0.24 | 0.00 | 0.00 | 0.00 |
| | Value 3 | 0.00 | 0.00 | 0.00 | 0.00 | 0.00 | 0.30 | 0.00 | 0.00 |
| | **Mean value** | **0.00** | **0.04** | **0.03** | **0.13** | **0.08** | **0.10** | **0.01** | **0.04** |

**Example 11 *In vivo* experiment**

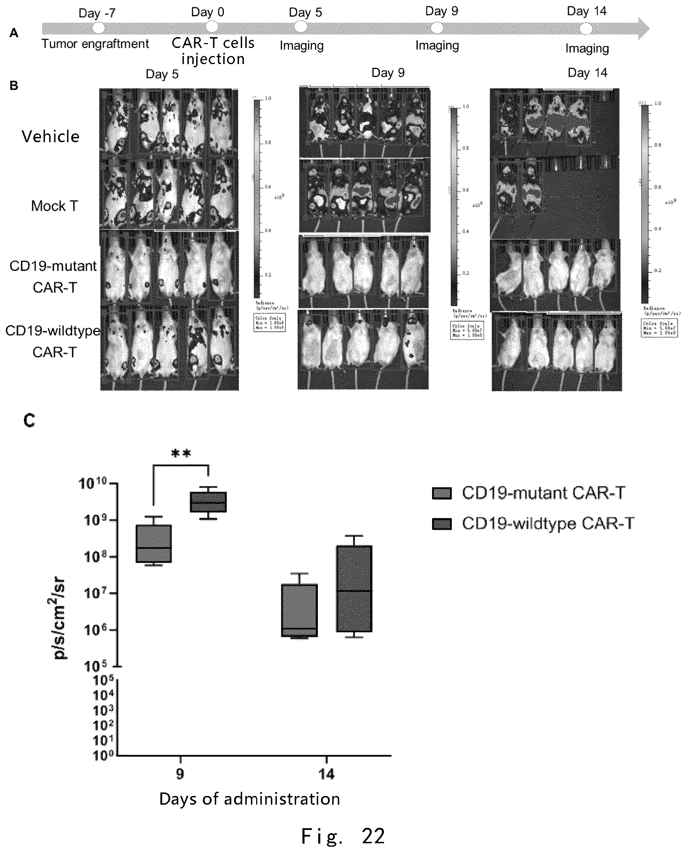

6-8-week-old B-NDG mice were selected and injected intravenously with tumor cells (Day -7). One day later (Day -6), the tumor graft load was measured. On Day 0, the mice were grouped, and after grouping, CAR-T cells were injected intravenously. After CAR-T treatment, the tumor load in the mice was assessed on the designated dates (Fig. 22(A), Fig. 23(A), Fig. 24(A) and Fig. 25(A)). Each mouse was intraperitoneally injected with 0.3 mg of D-luciferin (Yeasen Biotechnology), and after 4 minutes, imaging was performed under isoflurane anesthesia using the Xenogen IVIS imaging system (Perkin Elmer Life Sciences) with a 30-second exposure. Bioluminescence signals were calculated based on the emitted photon count, and the photon count was normalized to exposure time and surface area, resulting in photon count/s/cm$^2$/sr (p/s/cm$^2$/sr).

The *in vivo* pharmacodynamic test data in Fig. 22 show that, in the pharmacodynamic test on Nalm6-luc tumor-bearing model (tumor cell inoculated at 0.4E+6 Nalm6-luc cells/mouse), at different detection time points, a dose of 1.50E+6 cells/mouse of both CD19-mutant CAR-T and CD19-wildtype CAR-T demonstrated tumor-killing effects. On Day 5 and Day 9, CD19-mutant CAR-T cells cleared tumors more effectively than CD19-wildtype CAR-T cells. As shown in Fig. 22(B) and Fig. 22(C), on Day 9, CD19-mutant CAR-T cells completely cleared the tumor, and no recurrence was observed until Day 14. On Day 14, CD19-wildtype CAR-T cells completely cleared the tumor. After 14 days of treatment, one mouse in the vehicle group died; three mice in the Mock T group (no CAR transfection) died, while no deaths were observed in other groups. This indicates that the mutation in the co-stimulatory domain CD28 enhances the anti-tumor effect of CD19 CAR. During the experiment, the mice in the vehicle group and Mock T group showed significant weight loss, while no weight-related side effects were observed in the CD19-mutant CAR-T and CD19-wildtype CAR-T groups (Table 14). Therefore, the CD19-mutant CAR and CD19-wildtype CAR were combined with BCMA CAR to prepare BCMA and CD19 dual-target CAR-T cells to further confirm whether the mutation in the co-stimulatory domain CD28 enhances the anti-tumor effect of dual CAR-T cells.

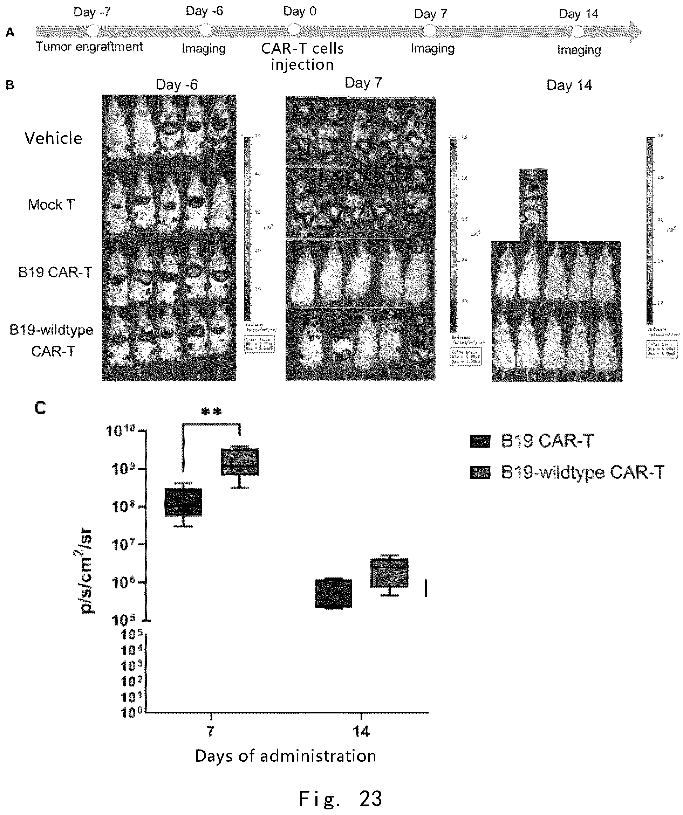

The *in vivo* pharmacodynamic test data in Fig. 23 show that, in the pharmacodynamic test on Nalm6-luc tumor-bearing model (tumor cell inoculated at 0.4E+6 Nalm6-luc cells/mouse), at different detection time points, a dose of 3.00E+6 cells/mouse of both B19 CAR-T cells and B19-wildtype CAR-T cells demonstrated tumor-killing effects. As shown in Fig. 23(B) and Fig. 23(C), on Day 7, B19 CAR-T cells cleared tumors more effectively than B19-wildtype CAR-T cells. B19 CAR-T cells almost completely cleared the tumor, while B19-wildtype CAR-T cells can not completely clear the tumor. On Day 14, B19 CAR-T and B19-wildtype CAR-T cells completely cleared the tumor. After 14 days of treatment, five mice in the vehicle group died; four mice in the Mock T group (no CAR transfection) died, while no deaths were observed in other groups. During the experiment, the mice in the vehicle group, Mock T group and B19-wildtype CAR-T group showed significant weight loss, while no weight-related side effects were observed in the B19 CAR-T groups (Table 15). This indicates that the mutation in the co-stimulatory domain CD28 enhances the killing effect against CD19+ tumor cells and improves the anti-tumor effect of B19 CAR-T, while reducing the side effects of CAR-T cells.

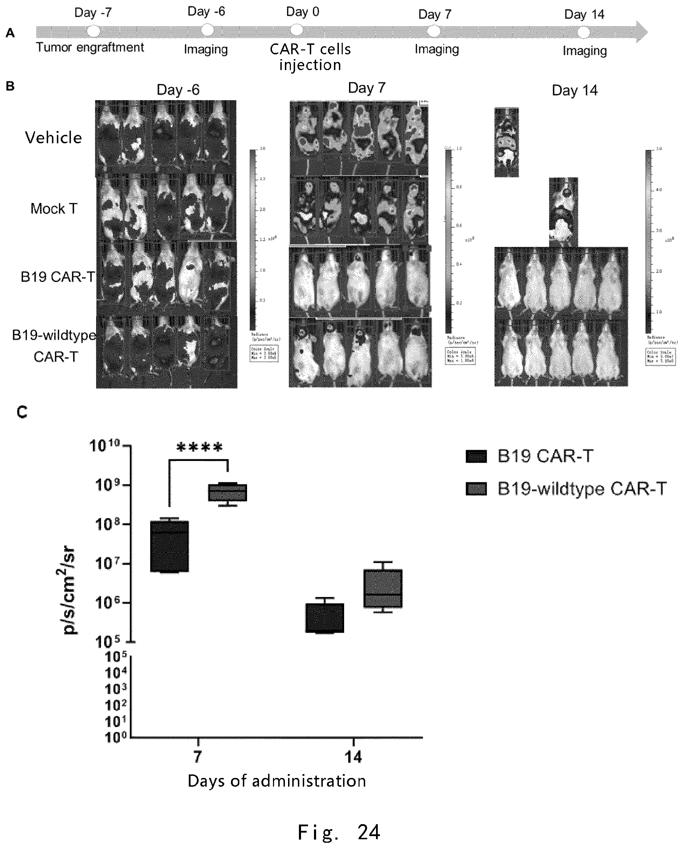

The *in vivo* pharmacodynamic test data in Fig. 24 show that, in the pharmacodynamic test on Nalm6-luc+H929-luc tumor-bearing model (tumor cell inoculated at 0.4E+6 Nalm6-luc cells and 5E+6 H929-luc cells/mouse), at different detection time points, a dose of 3.00E+6 cells/mouse of both B19 CAR-T cells and B19-wildtype CAR-T cells demonstrated tumor-killing effects. As shown in Fig. 24(B) and Fig. 24(C), on Day 7, B19 CAR-T cells cleared tumors more effectively than B19-wildtype CAR-T cells. B19 CAR-T cells almost completely cleared the tumor, while B19-wildtype CAR-T cells can not completely clear the tumor. On Day 14, B19 CAR-T and B19-wildtype CAR-T cells completely cleared the tumor. After 14 days of treatment, four mice in the vehicle group died; four mice in the Mock T group (no CAR transfection) died, while no deaths were observed in other groups. During the experiment, the mice in the vehicle group, Mock T group and B19-wildtype CAR-T group showed significant weight loss, while no weight-related side effects were observed in the B19 CAR-T groups (Table 16). This indicates that the mutation in the co-stimulatory domain CD28 enhances the killing effect against CD19+BCMA+ tumor cells and improves the anti-tumor effect of B19 CAR-T, while reducing the side effects of CAR-T cells.

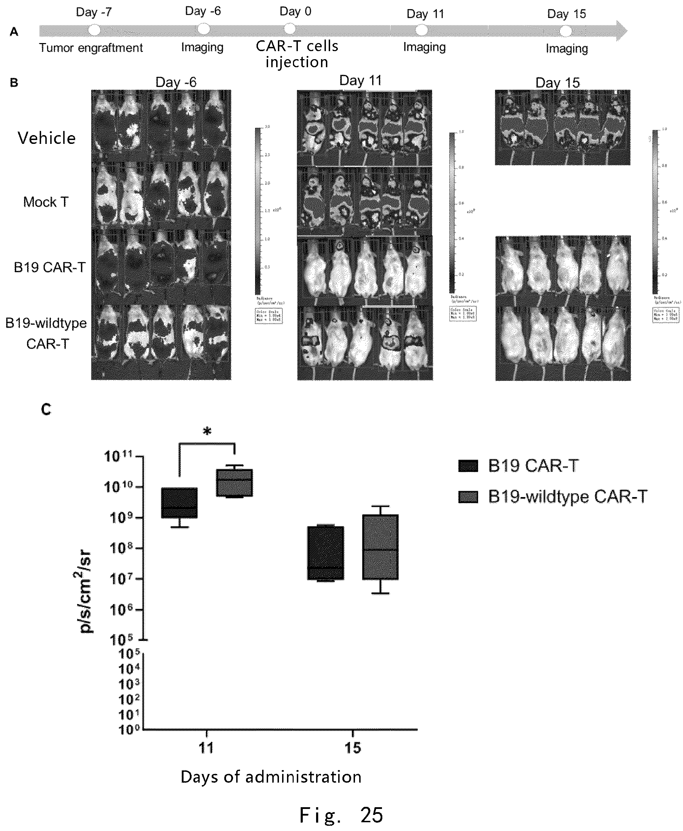

The *in vivo* pharmacodynamic test data in Fig. 25 show that, in the pharmacodynamic test on Nalm6-luc+H929-luc tumor-bearing model (tumor cell inoculated at 0.4E+6 Nalm6-luc cells and 5E+6 H929-luc cells/mouse), at different detection time points, a dose of 1.50E+6 cells/mouse of both B19 CAR-T cells and B19-wildtype CAR-T cells demonstrated tumor-killing effects. As shown in Fig. 25(B) and Fig. 25(C), on Day 7, B19 CAR-T cells cleared tumors more effectively than B19-wildtype CAR-T cells. B19 CAR-T cells almost completely cleared the tumor, while B19-wildtype CAR-T cells can not completely clear the tumor. On Day 14, B19 CAR-T and B19-wildtype CAR-T cells completely cleared the tumor. After 14 days of treatment, 5 mice in the Mock T group (no CAR transfection) died, while no deaths were observed in other groups. During the experiment, the mice in the vehicle group and Mock T group showed significant weight loss, while no weight-related side effects were observed in the B19 CAR-T and B19-wildtype CAR-T groups (Table 17). This indicates that the mutation in the co-stimulatory domain CD28 enhances the killing effect against CD19+BCMA+ tumor cells and improves the anti-tumor effect of B19 CAR-T.

The *in vivo* pharmacodynamic tests in Figs. 22-25 show that the mutation in the co-stimulatory domain CD28 enhances the tumor-killing effect of B19 CAR-T against Nalm6-luc and Nalm6-luc+H929-luc tumor cells. At the dose of 3.00E+6 cells/mouse, those in the B19 CAR-T group did not show significant weight loss compared with the mice in the B19-wildtype CAR-T treatment group, indicating that the mutation in the co-stimulatory domain CD28 improves the safety of B19 CAR-T.

In conclusion, the mutation in the co-stimulatory domain CD28 unexpectedly enhances the anti-tumor effects of both single-target CD19-mutant CAR-T cells and dual-target B19 CAR-T cells in mice, while improving the safety of B19 CAR-T.

**Table 14**

| Treatment Group | Number of Animal/No | Body Weight(g) | | | | | |
|---|---|---|---|---|---|---|---|
| | | 0 | 3 | 5 | 7 | 10 | 12 |
| Vehicle i.v. | 1150 | 20.3 | 21.1 | 20.0 | 20.1 | 17.5 | 18.10 |
| | 1170 | 20.2 | 21.0 | 19.8 | 19.5 | 16.7 | 16.20 |
| | 1174 | 21.7 | 22.4 | 19.5 | 19.5 | 17.9 | 17.50 |
| | 1180 | 22.0 | 23.4 | 20.7 | 21.0 | 18.9 | 17.90 |
| | 1185 | 23.4 | 23.0 | 21.9 | 22.1 | 19.2 | 18.20 |
| | **Mean value** | **21.5** | **22.18** | **20.38** | **20.44** | **18.04** | **17.58** |
| Mock T i.v. | 1156 | 20.4 | 20.9 | 19.7 | 19.6 | 17.5 | 16.8 |
| | 1149 | 21.1 | 21.2 | 20.5 | 20.1 | 17.3 | 16.4 |
| | 1152 | 21.8 | 22.4 | 20.7 | 20.2 | 17.8 | 16.2 |
| | 1163 | 21.9 | 21.4 | 20.8 | 20.7 | 18.7 | 17 |
| | 1157 | 22.5 | 23.7 | 21.7 | 21.2 | 17.9 | 16 |
| | **Mean value** | **21.5** | **21.92** | **20.68** | **20.36** | **17.84** | **16.48** |
| mutant CAR-T (1.50E+6cells/mouse) i.v. | 1176 | 20.5 | 20.2 | 20.0 | 19.6 | 20.3 | 20.7 |
| | 1148 | 21.0 | 21.5 | 20.9 | 20.8 | 21.1 | 21.8 |
| | 1161 | 21.7 | 21.6 | 20.6 | 20.9 | 22.5 | 22.6 |
| | 1153 | 20.9 | 20.8 | 20.2 | 19.6 | 21.2 | 21.1 |
| | 1164 | 23.6 | 24.4 | 23.2 | 23.4 | 23.5 | 22.5 |
| | **Mean value** | **21.5** | **21.70** | **20.98** | **20.86** | **21.72** | **21.74** |
| wildtype CAR-T (1.50E+6cells/mouse) i.v. | 1179 | 20.5 | 21.2 | 20.0 | 20.0 | 20.8 | 21.6 |
| | 1173 | 21.3 | 21.6 | 19.5 | 20.0 | 20.8 | 20.5 |
| | 1171 | 21.6 | 22.4 | 20.7 | 20.7 | 23.1 | 23.1 |
| | 1167 | 22.0 | 22.2 | 20.0 | 19.7 | 21.9 | 22.3 |
| | 1187 | 22.1 | 23.0 | 21.3 | 21.7 | 22.5 | 22.8 |
| | **Mean value** | **21.5** | **22.08** | **20.30** | **20.42** | **21.82** | **22.06** |

**Table 15**

| Treatment Group | Number of Animal/ No. | Body Weight(g) | | | | | | |
|---|---|---|---|---|---|---|---|---|
| | | 0 | 3 | 5 | 7 | 10 | 12 | 14 |
| Vehicle i.v. | 928 | 19.1 | 19.3 | 19.0 | 18.70 | 17.3 | - | - |
| | 926 | 19.6 | 19.6 | 18.5 | 18.40 | 17.5 | - | - |
| | 935 | 20.2 | 20.1 | 19.8 | 18.40 | 18.4 | 16.4 | - |
| | 927 | 20.5 | 20.4 | 18.9 | 18.90 | 18.0 | 16.0 | - |
| | 938 | 21.2 | 20.3 | 19.8 | 18.80 | 17.9 | 16.2 | - |
| | **Mean value** | **20.1** | **19.94** | **19.20** | **18.64** | **17.82** | **16.20** | **-** |
| Mock T i.v. | 937 | 18.3 | 19.0 | 18.5 | 17.4 | 16.5 | 15.3 | - |
| | 933 | 19.9 | 20.8 | 19.6 | 18.7 | 17.5 | 16.5 | 15.2 |
| | 924 | 20.2 | 20.5 | 18.9 | 17.6 | 17.2 | 15.3 | - |
| | 929 | 20.6 | 21.3 | 20.3 | 19.6 | 18.8 | 16.6 | - |
| | 932 | 21.5 | 21.6 | 20.5 | 18.9 | 18.7 | 17.6 | - |
| | **Mean value** | **20.1** | **20.64** | **19.56** | **18.44** | **17.74** | **16.26** | **15.20** |
| B19CAR-T (3.00E+6cells/ mouse) 1.v. | 934 | 19.1 | 19.3 | 18.4 | 18.5 | 19.4 | 19.9 | 20.0 |
| | 930 | 19.7 | 19.9 | 19.5 | 19.8 | 20.8 | 20.7 | 20.4 |
| | 941 | 20.1 | 19.5 | 18.6 | 18.5 | 19.8 | 18.2 | 17.1 |
| | 943 | 20.8 | 21.4 | 21.6 | 20.5 | 21.1 | 21.6 | 21.7 |
| | 942 | 21.0 | 21.7 | 20.5 | 21.2 | 22.0 | 22.9 | 23.2 |
| | **Mean value** | **20.1** | **20.36** | **19.72** | **19.70** | **20.62** | **20.66** | **20.48** |
| B 19-wildtype BCMA CAR-T (3.00E+6cells/ mouse) i.v. | 925 | 18.7 | 19.7 | 18.3 | 18.3 | 19.6 | 20.9 | 20.4 |
| | 940 | 19.6 | 18.7 | 16.7 | 15.9 | 16.2 | 17.2 | 18.3 |
| | 936 | 19.9 | 20.0 | 19.0 | 17.9 | 18.2 | 19.5 | 20.5 |
| | 923 | 20.6 | 21.1 | 20.6 | 19.3 | 20.6 | 20.9 | 21.9 |
| | 931 | 21.1 | 22.1 | 20.3 | 19.0 | 18.0 | 20.4 | 22.0 |
| | **Mean value** | **20.0** | **20.32** | **18.98** | **18.08** | **18.52** | **19.78** | **20.62** |

**Table 16**

| Treatment Group | Number of Animal/N o. | Body Weight(g) | | | | | | |
|---|---|---|---|---|---|---|---|---|
| | | 0 | 3 | 5 | 7 | 10 | 12 | 14 |
| Vehicle i.v. | 907 | 18.4 | 20.1 | 18.1 | 17.90 | 16.7 | 15.7 | 13.6 |
| | 915 | 18.9 | 18.7 | 18.0 | 17.10 | 16.0 | 14.9 | - |
| | 916 | 19.1 | 20.0 | 18.7 | 18.50 | 16.9 | 15.5 | - |
| | 906 | 20.4 | 20.0 | 20.5 | 18.50 | 17.4 | - | - |
| | 904 | 20.9 | 21.7 | 20.8 | 19.40 | 19.0 | 16.4 | - |
| | **Mean value** | **19.5** | **20.11** | **19.22** | **18.28** | **17.20** | **15.63** | **13.60** |

(continued)

| Treatment Group | Number of Animal/N o. | Body Weight(g) | | | | | | |
|---|---|---|---|---|---|---|---|---|
| | | 0 | 3 | 5 | 7 | 10 | 12 | 14 |
| Mock T i.v. | 912 | 18.1 | 18.6 | 17.5 | 17.7 | 14.1 | - | - |
| | 918 | 18.7 | 19.1 | 19.7 | 19.1 | 18.3 | 15.6 | - |
| | 919 | 19.1 | 19.8 | 18.9 | 18.4 | 17.6 | 16.8 | 15.4 |
| | 917 | 19.5 | 19.3 | 19.1 | 17.7 | 17.1 | 17.1 | 16.5 |
| | 921 | 22.8 | 21.7 | 21.3 | 19.4 | 18.7 | 17.0 | - |
| | **Mean value** | **19.6** | **19.70** | **19.30** | **18.46** | **17.16** | **16.63** | **15.95** |
| B19CAR-T (3.00E+6cells/m ouse) i.v. | 909 | 18.1 | 18.7 | 19.4 | 18.4 | 19.0 | 19.0 | 19.3 |
| | 914 | 19.1 | 19.6 | 19.6 | 20.2 | 19.1 | 19.6 | 19.4 |
| | 902 | 19.2 | 20.5 | 20.7 | 19.2 | 20.6 | 21.9 | 21.7 |
| | 910 | 19.8 | 20.0 | 18.8 | 19.3 | 20.6 | 21.4 | 21.0 |
| | 913 | 22.0 | 21.9 | 21.7 | 22.3 | 23.5 | 23.0 | 23.8 |
| | **Mean value** | **19.6** | **20.14** | **20.04** | **19.88** | **20.56** | **20.98** | **21.04** |
| B 19-wildtype BCMA CAR-T (3.00E+6cells/m ouse) i.v. | 905 | 18.3 | 18.4 | 16.7 | 16.3 | 14.8 | 15.4 | 14.6 |
| | 901 | 19.1 | 19.9 | 18.3 | 18.4 | 19.0 | 20.0 | 20.0 |
| | 908 | 19.5 | 20.1 | 17.9 | 18.5 | 19.5 | 21.2 | 22.0 |
| | 900 | 20.3 | 20.6 | 19.8 | 20.2 | 20.2 | 20.2 | 20.4 |
| | 903 | 20.7 | 21.9 | 21.4 | 20.8 | 21.9 | 22.6 | 22.8 |
| | **Mean value** | **19.6** | **20.18** | **18.82** | **18.84** | **19.08** | **19.88** | **19.96** |

**Table 17**

| Treatment Group | Number of Animal/No. | Body Weight(g) | | | |
|---|---|---|---|---|---|
| | | 0 | 3 | 5 | 7 |
| Vehicle i.v. | 1131 | 21.1 | 19.2 | 18.0 | 18.3 |
| | 1129 | 19.2 | 17.0 | 16.5 | 16.4 |
| | 1135 | 18.1 | 17.9 | 17.4 | 17.3 |
| | 1146 | 22.3 | 17.8 | 16.6 | 16.8 |
| | 1144 | 21.0 | 19.1 | 17.2 | 17.6 |
| | **Mean value** | **20.3** | **18.20** | **17.14** | **17.28** |
| Mock T i.v. | 1125 | 22.6 | 19.6 | - | - |
| | 1139 | 18.4 | 17.1 | 15.7 | 15.7 |
| | 1136 | 21.1 | 21.2 | 18.9 | - |
| | 1145 | 18.4 | 16.8 | 15.8 | 15.5 |
| | 1142 | 21.5 | 18.2 | 18.1 | - |
| | **Mean value** | **20.4** | **18.58** | **17.13** | **15.60** |

(continued)

| Treatment Group | Number of Animal/No. | Body Weight(g) | | | |
|---|---|---|---|---|---|
| | | 0 | 3 | 5 | 7 |
| B19CAR-T (1.50E+6cells/mouse) i.v. | 1128 | 19.5 | 18.2 | 20.0 | 20.2 |
| | 1141 | 21.0 | 18.9 | 18.3 | 19.2 |
| | 1137 | 19.3 | 19.7 | 21.1 | 21.0 |
| | 1130 | 20.2 | 19.3 | 20.3 | 20.6 |
| | 1143 | 21.7 | 18.5 | 20.1 | 19.2 |
| | **Mean value** | **20.3** | **18.92** | **19.96** | **20.04** |
| B19-wildtype BCMA CAR-T (1.50E+6cells/mouse) i.v. | 1127 | 21.2 | 19.4 | 18.9 | 19.2 |
| | 1126 | 20.0 | 19.3 | 19.7 | 20.6 |
| | 1133 | 22.0 | 20.4 | 20.6 | 22.5 |
| | 1132 | 18.6 | 18.8 | 16.2 | 16.3 |
| | 1138 | 20.0 | 21.2 | 19.8 | 20.8 |
| | **Mean value** | **20.4** | **19.82** | **19.04** | **19.88** |

**Table 18 Sequence listing**

| Sequence | Combination | Amino acid sequence |
|---|---|---|
| 1 | CSF2R signal peptide | MLLLVTSLLLCELPHPAFLLIP |
| 2 | BCMA scFv | DIVLTQSPPSLAMSLGKRATISCRASESVTILGSHLIHWYQQKPGQPPTLLIQLASNVQTGVPARFSGSGSRTDFTLTIDPVEEDDVAVYYCLQSRTIPRTFGGGTKLEIKGGGGSGGGGSGGGGSQIQLVQSGPELKKPGETVKISCKASGYTFTDYSINWVKRAPGKGLKWMGWINTETREPAYAYDFRGRFAFSLETSASTAYLQINNLKYEDTATYFCALDYSYAMDYWGQGTSVTVSS |
| 3 | CD8 hinge region | TTTPAPRPPTPAPTIASQPLSLRPEACRPAAGGAVHTRGLDFACDIY |
| 4 | CD28 transmembrane region | IWAPLAGTCGVLLLSLVITLYC |
| 5 | 4-1BB co-stimulatory domain | KRGRKKLLYIFKQPFMRPVQTTQEEDGCSCRFPEEEEGGCEL |
| 6 | CD3ζ | RVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 7 | T2A | EGRGSLLTCGDVEENPGP |
| 8 | CD8 signal peptide | MALPVTALLLPLALLLHAARP |

(continued)

| Sequence | Combination | Amino acid sequence |
|---|---|---|
| 9 | CD19 scFv | DIQMTQTTSSLSASLGDRVTISCRASQDISKYLNWYQQKPDGTVKLLIYHTSRLHSGVPSRFSGSGSGTDYSLTISNLEQEDIATYFCQQGNTLPYTFGGGTKLEITGGGGSGGGGSGGGGSEVKLQESGPGLVAPSQSLSVTCTVSGVSLPDYGVSWIRQPPRKGLEWLGVIWGSETTYYNSALKSRLTIIKDNSKSQVFLKMNSLQTDDTAIYYCAKHYYYGGSYAMDYWGQGTSVTVSS |
| 10 | Linking peptide | GGGGSGGGGSGGGGS |
| 11 | CD28 hinge-transmembrane domain-CD28( YMFM) | IEVMYPPPYLDNEKSNGTIIHVKGKHLCPSPLFPGPSKPFWVLVVVGGVLACYSLLVTVAFIIFWVRSKRSRLLHSDYMFMTPRRPGPTRKHYQPYAPPRDFAAYRS |
| 12 | CD28 hinge-transmembrane domain-CD28-YMNM | IEVMYPPPYLDNEKSNGTIIHVKGKHLCPSPLFPGPSKPFWVLVVVGGVLACYSLLVTVAFIIFWVRSKRSRLLHSDYMNMTPRRPGPTRKHYQPYAPPRDFAAYRS |
| 13 | CD28-YMNM | RSKRSRLLHSDYMNMTPRRPGPTRKHYQPYAPPRDFAAYRS |
| 14 | CD28-YMFM | RSKRSRLLHSDYMFMTPRRPGPTRKHYQPYAPPRDFAAYRS |
| 15 | BCMA CAR-T | MLLLVTSLLLCELPHPAFLLIPDIVLTQSPPSLAMSLGKRATISCRASESVTILGSHLIHWYQQKPGQPPTLLIQLASNVQTGVPARFSGSGSRTDFTLTIDPVEEDDVAVYYCLQSRTIPRTFGGGTKLEIKGGGGSGGGGSGGGSQIQLVQSGPELKKPGETVKISCKASGYTFTDYSINWVKRAPGKGLKWMGWINTETREPAYAYDFRGRFAFSLETSASTAYLQINNLKYEDTATYFCALDYSYAMDYWGQGTSVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRGKGHDGLYQGLSTATKDTYDALHMQALPPR |

(continued)

| Sequence | Combination | Amino acid sequence |
|---|---|---|
| 16 | mutant CAR-T | MALPVTALLLPLALLLHAARPDIQMTQTTSSLSAS LGDRVTISCRASQDISKYLNWYQQKPDGTVKLLIY HTSRLHSGVPSRFSGSGSGTDYSLTISNLEQEDIAT YFCQQGNTLPYTFGGGTKLEITGGGGSGGGGSGG GGSEVKLQESGPGLVAPSQSLSVTCTVSGVSLPDY GVSWIRQPPRKGLEWLGVIWGSETTYYNSALKSR LTIIKDNSKSQVFLKMNSLQTDDTAIYYCAKHYYY GGSYAMDYWGQGTSVTVSSIEVMYPPPYLDNEKS NGTIIHVKGKHLCPSPLFPGPSKPFWVLVVVGGVL ACYSLLVTVAFIIFWVRSKRSRLLHSDYMFMTPRR PGPTRKHYQPYAPPRDFAAYRSRVKFSRSADAPA YQQGQNQLYNELNLGRREEYDVLDKRRGRDPEM GGKPRRKNPQEGLYNELQKDKMAEAYSEIGMKG ERRRGKGHDGLYQGLSTATKDTYDALHMQALPP R |
| 17 | wildtype CAR-T | MALPVTALLLPLALLLHAARPDIQMTQTTSSLSAS LGDRVTISCRASQDISKYLNWYQQKPDGTVKLLIY HTSRLHSGVPSRFSGSGSGTDYSLTISNLEQEDIAT YFCQQGNTLPYTFGGGTKLEITGGGGSGGGGSGG GGSEVKLQESGPGLVAPSQSLSVTCTVSGVSLPDY GVSWIRQPPRKGLEWLGVIWGSETTYYNSALKSR LTIIKDNSKSQVFLKMNSLQTDDTAIYYCAKHYYY GGSYAMDYWGQGTSVTVSSIEVMYPPPYLDNEKS NGTIIHVKGKHLCPSPLFPGPSKPFWVLVVVGGVL ACYSLLVTVAFIIFWVRSKRSRLLHSDYMNMTPRR PGPTRKHYQPYAPPRDFAAYRSRVKFSRSADAPA YQQGQNQLYNELNLGRREEYDVLDKRRGRDPEM GGKPRRKNPQEGLYNELQKDKMAEAYSEIGMKG ERRRGKGHDGLYQGLSTATKDTYDALHMQALPP R |

In SEQ ID NO. 2 and SEQ ID NO. 9, the light variable region of scFv is indicated with single-underlined, while the heavy variable region of scFv is indicated with double-underline.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

**Claims**

1. An engineered immune cell which expresses a BCMA CAR and a CD19-mutant CAR in parallel, wherein the amino acid sequence of the BCMA CAR is as shown in SEQ ID NO: 15; the amino acid sequence of the CD19-mutant CAR is as shown in SEQ ID NO: 16; wherein the intracellular co-stimulatory domain of the CD19-mutant CAR comprises a CD28 mutant as shown in SEQ ID NO: 14, and the wild-type motif YMNM is mutated to YMFM in the CD28 mutant.

2. A method for preparing the engineered immune cell according to claim 1, comprising steps of:

    (A) providing an immune cell to be engineered; and

(B) engineering the immune cell such that the immune cell expresses the BCMA CAR and CD19-mutant CAR, thereby obtaining an engineered immune cell according to claim 1.

3. A fusion protein which comprises a BCMA CAR sequence and a CD19-mutant CAR sequence connected by a self-cleaving sequence, wherein the amino acid sequence of the BCMA CAR is as shown in SEQ ID NO: 15; the amino acid sequence of the CD19-mutant CAR is as shown in SEQ ID NO: 16; wherein the intracellular co-stimulatory domain of the CD19-mutant CAR comprises a CD28 mutant as shown in SEQ ID NO: 14, and the wild-type motif YMNM is mutated to YMFM in the CD28 mutant.

4. A polynucleotide encoding the fusion protein according to claim 3.

5. A vector comprising the polynucleotide according to claim 4.

6. A pharmaceutical composition comprising the engineered immune cell according to claim 1, and a pharmaceutically acceptable carrier, diluent or excipient.

7. Use of the engineered immune cell according to claim 1 or the pharmaceutical composition according to claim 6 in the preparation of a medicament or formulation for preventing and/or treating a disease, wherein the disease is selected from a group consisting of a hematological tumor, a solid tumor, an autoimmune disease or a combination thereof.

BCMA CAR

| CSF2R SP | BCMA-scFv | CD8 H+TM | 4-1BB | CD3ζ |
|----------|-----------|----------|-------|------|

CD19-mutant CAR

| CD8 SP | CD19-scFv | CD28 H+TM | **CD28-YMFM** | CD3ζ |
|--------|-----------|-----------|---------------|------|

| Promoter | BCMA CAR | | CD19-mutant CAR |
|----------|----------|--|-----------------|

T2A

The structure of B19 CAR

BCMA CAR

| CSF2R SP | BCMA-scFv | CD8 H+TM | 4-1BB | CD3ζ |
|----------|-----------|----------|-------|------|

CD19-wildtype CAR

| CD8 SP | CD19-scFv | CD28 H+TM | **CD28-YMNM** | CD3ζ |
|--------|-----------|-----------|---------------|------|

| Promoter | BCMA CAR | | CD19-wildtype CAR |
|----------|----------|--|-------------------|

T2A

The structure of B19-wildtype CAR

CD19-mutant CAR

| CD8 SP | CD19-scFv | CD28 H+TM | **CD28-YMFM** | CD3ζ |
|--------|-----------|-----------|---------------|------|

| Promoter | CD19-mutant CAR |
|----------|-----------------|

The structure of CD19-mutant CAR

CD19-wildtype CAR

| CD8 SP | CD19-scFv | CD28 H+TM | **CD28-YMNM** | CD3ζ |
|--------|-----------|-----------|---------------|------|

| Promoter | CD19-wildtype CAR |
|----------|-------------------|

The structure of CD19-wildtype CAR

BCMA CAR

| CSF2R SP | BCMA-scFv | CD8 H+TM | 4-1BB | CD3ζ |
|----------|-----------|----------|-------|------|

| Promoter | BCMA CAR |
|----------|----------|

The structure of BCMA CAR

# Fig. 1

Fig. 2

Fig. 3

**CD19-mutant CAR plasmid map**
8753 bp

Fig. 4

**CD19-wildtype CAR plasmid map**
8753 bp

Fig. 5

**BCMA CAR plasmid map**
8771 bp

lac promoter
RSV promoter
5' LTR (truncated)
HIV-1 ψ
RRE
cPPT/CTS
EF-1α promoter
EF-1α intron A
CSF2R signal peptide
BCMV-scFv
CD8 hinge
CD8 α TM
4-1BB
CD3ζ
WPRE
Factor Xa site
SV40 poly(A) signal
SV40 ori
f1 ori
KanR
ori

Fig. 6

Fig. 7

Fig. 8

Fig. 9

Fig. 10

Fig. 11

Fig. 12

Fig. 13A

Fig. 13B

Fig. 14

Fig. 15

Fig. 16

Fig. 17

Fig. 18

F i g. 19

Fig. 20A

Fig. 20B

Fig. 21A

Fig. 21B

Fig. 22

Fig. 23

Fig. 24

Fig. 25

## INTERNATIONAL SEARCH REPORT

| | International application No. |
|---|---|
| | **PCT/CN2024/084111** |

**A. CLASSIFICATION OF SUBJECT MATTER**

C12N 5/10(2006.01)i; C07K 16/28(2006.01)i; C07K 19/00(2006.01)i; C12N 15/62(2006.01)i; C12N 15/867(2006.01)i; C12N 5/09(2010.01)i; A61K 39/00(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

IPC:C12N,C07K,A61K,A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, 超星读秀学术, DUXIU ACADEMIC,中国专利生物序列检索系统, CHINA PATENTS BIOLOGICAL SEQUENCE DATABASE, CNKI, 万方, WANFANG, ISI web of knowledge, Elsevier Science Direct, Springerlink, pubmed, genbank, ENA, EMBL, Uniport, STN, 百度, BAIDU, 百度学术, BAIDU SCHOLAR: BCMA, CD19, 抗体, CAR-T细胞, 嵌合抗原受体, CAR, 并联, 共刺激域, CD28, 基序, SEQ ID NO.14-16

**C. DOCUMENTS CONSIDERED TO BE RELEVANT**

| Category* | Citation of document, with indication, where appropriate, of the relevant passages | Relevant to claim No. |
|---|---|---|
| PX | CN 116478929 A (SHANGHAI KEQI PHARMACEUTICAL TECHNOLOGY CO., LTD.) 25 July 2023 (2023-07-25) claims 1-10 | 1-7 |
| X | CN 112592927 A (SHANGHAI KEQI PHARMACEUTICAL TECHNOLOGY CO., LTD.) 02 April 2021 (2021-04-02) claims 1-18, and description, paragraphs 46-50, and table 1 and table 2 | 1-7 |
| A | WO 2020061796 A1 (HRAIN BIOTECHNOLOGY CO., LTD.) 02 April 2020 (2020-04-02) claims 1-9 | 1-7 |
| A | CN 115715298 A (SICHUAN KELUN-BIOTECH BIOPHARMACEUTICAL CO., LTD.) 24 February 2023 (2023-02-24) claims 1-23 | 1-7 |
| A | CN 111511370 A (JUNO THERAPEUTICS, INC.) 07 August 2020 (2020-08-07) sequence 328 | 1-7 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 June 2024** | **20 June 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/084111** |

**C.    DOCUMENTS CONSIDERED TO BE RELEVANT**

| Category* | Citation of document, with indication, where appropriate, of the relevant passages | Relevant to claim No. |
|---|---|---|
| A | CN 115515983 A (GRACELL BIOTECHNOLOGY (SHANGHAI) CO., LTD.) 23 December 2022 (2022-12-23)<br>claims 1-15 | 1-7 |
| A | CN 113784733 A (GRACELL BIOTECHNOLOGY (SHANGHAI) CO., LTD.) 10 December 2021 (2021-12-10)<br>claims 1-12 | 1-7 |
| A | CN 112566643 A (REGENTS OF THE UNIVERSITY OF CALIFORNIA) 26 March 2021 (2021-03-26)<br>claims 15-16 | 1-7 |
| A | CN 113286811 A (UNIVERSITY OF SOUTHERN CALIFORNIA) 20 August 2021 (2021-08-20)<br>description, paragraph 435 | 1-7 |

Form PCT/ISA/210 (second sheet) (July 2022)

**INTERNATIONAL SEARCH REPORT**

International application No.

**PCT/CN2024/084111**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT
### Information on patent family members

International application No.

**PCT/CN2024/084111**

| Patent document cited in search report | | | Publication date (day/month/year) | Patent family member(s) | | | Publication date (day/month/year) |
|---|---|---|---|---|---|---|---|
| CN | 116478929 | A | 25 July 2023 | None | | | |
| CN | 112592927 | A | 02 April 2021 | None | | | |
| WO | 2020061796 | A1 | 02 April 2020 | JP | 2022501067 | A | 06 January 2022 |
| CN | 115715298 | A | 24 February 2023 | US | 2023203178 | A1 | 29 June 2023 |
| | | | | WO | 2022007650 | A1 | 13 January 2022 |
| CN | 111511370 | A | 07 August 2020 | KR | 20200074997 | A | 25 June 2020 |
| | | | | EP | 3703688 | A2 | 09 September 2020 |
| | | | | JP | 2023082109 | A | 13 June 2023 |
| | | | | MA | 49911 | A | 24 June 2020 |
| | | | | AU | 2018358067 | A1 | 07 May 2020 |
| | | | | JP | 2021501605 | A | 21 January 2021 |
| | | | | JP | 7256197 | B2 | 11 April 2023 |
| | | | | US | 2023365699 | A1 | 16 November 2023 |
| | | | | WO | 2019089969 | A2 | 09 May 2019 |
| | | | | WO | 2019089969 | A3 | 20 June 2019 |
| | | | | CA | 3080904 | A1 | 09 May 2019 |
| | | | | IL | 274147 | A | 30 June 2020 |
| | | | | SG | 11202003501 | XA | 28 May 2020 |
| | | | | US | 2020392236 | A1 | 17 December 2020 |
| | | | | US | 11623961 | B2 | 11 April 2023 |
| | | | | BR | 112020008323 | A2 | 03 November 2020 |
| | | | | MX | 2020004243 | A | 25 September 2020 |
| CN | 115515983 | A | 23 December 2022 | TW | 202144434 | A | 01 December 2021 |
| | | | | TWI | 797610 | B | 01 April 2023 |
| | | | | WO | 2021223720 | A1 | 11 November 2021 |
| | | | | US | 2023235052 | A1 | 27 July 2023 |
| CN | 113784733 | A | 10 December 2021 | EP | 3964238 | A1 | 09 March 2022 |
| | | | | EP | 3964238 | A4 | 14 September 2022 |
| | | | | WO | 2020224605 | A1 | 12 November 2020 |
| | | | | TW | 202108620 | A | 01 March 2021 |
| | | | | TWI | 771678 | B | 21 July 2022 |
| | | | | US | 2022202864 | A1 | 30 June 2022 |
| CN | 112566643 | A | 26 March 2021 | CA | 3103610 | A1 | 19 December 2019 |
| | | | | EP | 3806871 | A2 | 21 April 2021 |
| | | | | EP | 3806871 | A4 | 23 February 2022 |
| | | | | US | 2021230289 | A1 | 29 July 2021 |
| | | | | WO | 2019241358 | A2 | 19 December 2019 |
| | | | | WO | 2019241358 | A3 | 13 February 2020 |
| CN | 113286811 | A | 20 August 2021 | AU | 2019315440 | A1 | 18 February 2021 |
| | | | | EP | 3830112 | A1 | 09 June 2021 |
| | | | | EP | 3830112 | A4 | 21 December 2022 |
| | | | | WO | 2020028444 | A1 | 06 February 2020 |
| | | | | US | 2021290676 | A1 | 23 September 2021 |
| | | | | CA | 3107675 | A1 | 06 February 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5399346 A
- US 5580859 A
- US 5589466 A
- WO 0196584 A
- WO 0129058 A
- US 6326193 B
- US 5350674 A
- US 5585362 A

### Non-patent literature cited in the description

- **SAMBROOK et al.** Molecular Cloning: A Laboratory Manual. Cold Spring Harbor Laboratory, 2001
- **ROSENBERG et al.** *New Eng. J. of Med.*, 1988, vol. 319, 1676
- **SAMBROOK et al.** Molecular Cloning: Laboratory Manual. Cold Spring Harbor Laboratory Press, 1989